# EUROPÄISCHE PATENTSCHRIFT

(11) **EP 1 412 473 B1**
(45) Veröffentlichungstag und Bekanntmachung des Hinweises auf die Patenterteilung: **14.01.2015**
(21) Anmeldenummer: 01964867.4
(22) Anmeldetag: 31.07.2001
(51) Int. Cl.: C12M 1/26

(54) **BIOREAKTOR MIT EINER APPARATUR MIT FLEXIBLEN WANDUNGEN**
BIOREACTOR PROVIDED WITH EQUIPMENT WITH FLEXIBLE WALLS
BIOREACTEUR COMPRENANT UN APPAREILLAGE A PAROIS SOUPLES

(43) Veröffentlichungstag der Anmeldung: 28.04.2004
(73) Patentinhaber: Sartorius Stedim Biotech GmbH, 37079 Göttingen (DE)
(72) Erfinder: Erhardt, Ursula, 10629 Berlin (DE); Erhardt, Christoph, 10629 Berlin (DE)
(74) Vertreter: Müller-Boré & Partner Patentanwälte PartG mbB
(86) Internationale Anmeldenummer: PCT/DE2001/002901
(87) Internationale Veröffentlichungsnummer: WO 2003/012027

(56) Entgegenhaltungen:
- GB-A- 2 182 647
- US-A- 4 665 035
- PATENT ABSTRACTS OF JAPAN vol. 018, no. 648 (C-1284), 8. Dezember 1994 (1994-12-08) & JP 06 253815 A (BIO POLYMER RES:KK), 13. September 1994 (1994-09-13)

## Beschreibung

### Gebiet der Erfindung.

Die Erfindung betrifft einen Bioreaktor bzw. ein Kulturgefäß zur Verwendung in der Kultivierung von Mikroorganismen, Zellen oder zellfreien Expressionssystemen. Bioreaktoren werden in Bereichen verwendet, in denen in Kulturgefäßen biologische oder biochemische Reaktionen durchgeführt werden, speziell im Bereich Biotechnologie, Lebensmitteltechnik und Umweltschutz.

### Stand der Technik.

Die Fermentationstechnologie hat vorwiegend auf dem Gebiet der Kultivierung von (genneukombinierten) Mikroorganismen und Zellen in den letzten 20 Jahren erhebliche Fortschritte gemacht. Ziel ist, Produktionsverfahren und Verfahrensentwicklungen zur Kultivierung von Zellen und Mikroorganismen durch maximale Ausbeuten so kostengünstig wie möglich zu gestalten. Der limitierende Faktor ist dabei meist nicht der Gehalt des Nährmediums, sondern die technische Ausrüstung der Kulturgefäße, die den Nachschub und die Durchmischung von Gasen und Flüssigkeiten gewährleisten. Die Kultivierung beginnt meist mit einer Beimpfung von kleinen Kulturflaschen mit 10 ml Maßstab und wird in Anlagen bis 1000 m³ mit hoher technischer Ausrüstung weitergeführt. Die Verfahrensentwicklung, auch für große Anlagen wird aus Kostengründen in kleinen Kulturflaschen soweit wie möglich betrieben. Die Verfahrensentwicklung in kleinen Kulturgefäßen betrifft jedoch meist nur die Optimierung der Medienbestandteile, nicht der Mengen- und Nachfütterintervalle, da die Kulturgefäße und deren technische Ausrüstung sehr stark differieren. Die Kulturbedingungen sind so unterschiedlich und keineswegs vergleichbar, daß beim Scale up in jedem Falle nachoptimiert werden muß.

Stand der Technik von Anlagen (Reaktoren) im Maßstab 0,001 m³ bis 1000 m³ Fermenter sind Reaktionskessel aus Glas oder meist Edelstahl im Maßstab von 1 Liter bis mehrere 100 Kubikmeter Arbeitsvolumen. Sie dienen im Produktionsmaßstab der kontrollierten Durchführung von biologischen oder biochemischen Reaktionen in der Biotechnologie, der Lebensmittelindustrie und der Abwasserreinigung. Es lassen sich drei wesentliche Bauelemente darstellen:

### a) der Reaktionsbehälter

Der Behälter ist meist ein Rührkessel mit eingebauter Rührwelle und Rührblättern, mit oder ohne Wirbelstrombrechern (Schikanen). Weiterhin kann die Durchmischung des Mediums über äußere oder innere Flüssigkeitsschlaufen erfolgen, die mit Luft oder Pumpen angetrieben werden. Die Begasung erfolgt über Luftausströmerrohre oder Luftausströmerringe. Messsonden werden über seitliche oder im Deckel angebrachte Gewindestutzen eingebracht.

### b) Versorgungstechnik

Über eine neben dem Reaktionsbehälter angebrachte Versorgungstechnik wird die Einhaltung der Reaktionsbedingungen gewährleistet. Hierzu zählen u.a. der Rührantrieb, die Temperierung, die Dosierstrecken für pH Regelung oder Substratdosierung und für die Regelung der Belüftung

### c) Mess- und Regeltechnik

In einem neben dem Reaktionsbehälter und der Versorgungstechnik angebrachten Schaltschrank befindet sich die Mess- und Regeltechnik oder ein EDV gestütztes Prozessleitsystem, das die Einhaltung der Reaktionsbedingungen im Reaktor gewährleistet und dokumentiert.

Um im Produktionsmaßstäb einen Fermenter wirtschaftlich betreiben zu können, müssen die optimalen Reaktionsbedingungen in einer Vielzahl von Vorversuchen ermittelt werden. Der hohe technische und wirtschaftliche Aufwand schließt den Einsatz von Fermentern für diese Vorversuche (z.B. Medienoptimierung) aus, so daß auf die nachfolgend beschriebenen Kulturgefäße, überwiegend geschüttelte Erlenmeyerkolben zurückgegriffen wird.

Aus der Praxis bekannter Stand der Technik Kulturgefäße im Labormaßstab ist folgender. Die für Mikroorganismen verwendeten Kulturgefäße werden im Maßstab 10 ml bis 5000ml Volumen unter temperierten Bedingungen entweder auf geeigneten Schüttelapparaturen geschüttelt oder auf Magnetrührern gerührt. Das neben einer Vielzahl von Kulturgefäßen am häufigsten verwendete Kulturgefäß ist der Erlenmeyerkolben (s. Katalog Fisher Scientific, S. 249 ff) im Maßstab 10 ml bis 1000 ml. Es handelt sich um reine 2 Phasensysteme, da eine Begasung des Kulturgefäßes nicht stattfindet. Durch die Kreisbewegung des Schüttlers bildet sich in der Reaktionsflüssigkeit eine meist laminare Kreisströmung aus, die eine nur mangelnde Durchmischung gewährleistet. Obwohl Erlenmeyerkolben mit im Glas eingepressten Wirbelstrom- brechern (Schikanen) verwendet werden können, um eine turbulente Mischung der Reaktionsflüssigkeit zu erzeugen, ist immer noch der Gasaustausch in der Reaktionsflüssigkeit eingeschränkt, da der Gasaüstausch nur an der Phasengrenzfläche zwischen Flüssigkeit und head space (darüber liegender Luft) diffusionskontrolliert erfolgen kann. Eine Zuführung der dritten Phase (Gas) fehlt. Die Effektivität gegenüber einem Dreiphasensystem liegt unter 1 Prozent. Die Nachlieferung von unverbrauchter Zuluft erfolgt durch Diffusion über statische Filtersysteme oder durch Diffusion unter einer Stahlkappe und ist somit ebenfalls ein limitierender Schritt. Ebensowenig existieren Möglichkeiten für eine geregelte Flüssigkeitendosierung oder das Einbringen von Messonden. Bedingt durch die Geometrie der Erlenmeyerkolben (Enghals) ist das Einbringen fester Einbauten in den Reaktionsraum stark eingeschränkt. Die Leistungen großer Reaktoren lassen sich nicht annähernd simulieren und definierte und reproduzierbare Bedingungen, wie sie zur Dokumentation der Reaktion oder für ein Scale up in große Reaktoren benötigt werden, lassen sich hiermit nicht erzielen.

Weiterhin verwendet werden sog. Blasensäulen in Form von Kulturflaschen (s. Katalog Fisher Scientific, S 248), bei denen der Glasboden durch eine poröse Platte ersetzt ist, durch die Luft in die Kulturflasche eingeblasen wird. Diese Art der Begasung (Dreiphasensystem) ist effektiver als das o.g. 2 Phasensystem, jedoch limitiert die Einblasrichtung (Blasen durchströmen die Flüssigkeit auf kürzeren Weg) nach oben auf Grund der kürzeren Kontaktzeit und der Förderung der Schaumbildung die Belüftungsrate, die fehlende Kreisbewegung durch Schütteln oder Rühren, sowie die fehlenden Wirbelstrombrecher (Schikanen) vermindern ebenfalls die Effektivität der Mischung der Reaktionsflüssigkeit (Infors Homepage www.infors.ch/d/d5a.htm). Strömungstechnisch sind Blasensäulen somit äußerst ungünstig, da häufig nur eine laminare Aufwärtsbewegung der Flüssigkeit erzeugt wird. Durch einen seitlichen Glasstutzen kann ggf. eine pH Sonde zur Messung des pH Wertes eingebracht werden, sowie eine Probenahme der Reaktionsflüssigkeit erfolgen.

Im Bereich der Kultivierung von Zellen werden gerührte "Spinner", d.h. Glasgefäße mit einem am Deckel eingehängten Magnetrührstab verwendet (s. Katalog Fisher Scientific, S. 251). Der Antrieb erfolgt über einen Magnetrührer, der unter dem Gefäß steht, von unten. Wirbelstrombrecher (Schikanen) oder Belüftungssysteme in Form von Einbauten sind nicht vorhanden und auf Grund der Geometrie, ähnlich wie beim Erlenmeyerkolben auch nicht möglich. Somit wird vom Rührstab nur eine äußerst uneffektive, häufig laminare Kreisströmung erzeugt mit dem Nachteil mangelnder Durchmischung. Durch einen seitlichen Glasstutzen kann ggf. eine pH Sonde zur Messung des pH Wertes eingebracht werden, sowie eine Probenahme der Reaktionsflüssigkeit erfolgen.

Die Verwendung von Kulturgefäßen weist zusammenfassend folgende Probleme und Nachteile auf:
- keine Vergleichbarkeit mit großen Fermentern
- keine optimale Versorgung mit Gasen oder Flüssigkeiten
- keine Dokumentation des Prozesses
- keine Reproduzierbarkeit

Mehrere Firmen (z. B. Infors AG, www.Infors.ch/d/d5a.htm) oder Das GIP GmbH, www.dasg@de) bieten neuerdings "Zwitterlösungen" an, bei denen eine Versorgungstechnik und eine EDV gestützte Mess- und Regeleinheit parallel mehrere Kleinfermenter oder bis zu 16 Kulturgefäße betreibt. Dies ermöglicht, den wirtschaftlichen und technischen Aufwand zu verkleinern und den Zeitraum für die Vorversuche durch paralleles Arbeiten zu verkleinern. Nachteilig ist auch hier, daß die Reaktionsbedingungen, insbesondere durch die Kulturgefäße, nicht mit den Reaktionsbedingungen der Fermenter verglichen werden können und so die Optimierung nur unzulänglich durchgeführt wird. Eine Sauerstoffeintragsrate, die im Fermenter durchaus 100 mal höher sein kann als im geschüttelten Erlenmeyerkolben führt zu vollkommen anderen Stoffwechselbedingungen des Organismus und macht die vorher optimierte Medienzusammensetzung und Produktionsrate zunichte.

US 4 665 035 A offenbart ein Kulturgefäß in dessen Inneren Lenkplatten angeordnet sind. Zur besseren Belüftung des Mediums ist das Kulturgefäß nach oben hin geöffnet. Auf die Öffnung kann ein gasdurchlässiger Filter angeordnet werden.

### Technisches Problem.

Der Erfindung liegt daher das technische Problem zu Grunde, einen Bioreaktor bzw. ein Kulturgefäss für den Labormassstab anzugeben, welcher optimale und reproduzierbare Kultivierungsergebnisse liefert und insbesondere leicht upscale-bar ist, i.e. ohne aufwendige Nachoptimierungen Bedingungen für den Produktionsmaßstab liefert.

Dieses Problem wird durch den Gegenstand der unabhängigen Ansprüche gelöst. Bevorzugte Ausführungsformen ergeben sich aus den abhängigen Ansprüchen.

### Grundzüge der Erfindung.

Die Erfindung lehrt zur Lösung dieses technischen Problems eine aufblasbare oder auffaltbare Apparatur, die in geschüttelte oder gerührte Kulturgefäße für biologische und (bio)chemische Reaktionen eingesetzt wird oder das Kulturgefäß selbst darstellt und über geeignete Vorrichtungen zum Erzeugen einer turbulenten Durchmischung und/oder zum Begasen und/oder zum Dosieren in dieses Kulturgefäß und/oder für die Fixierung von Messwertaufnehmern (Sondenarmatur) in dem Kulturgefäß verfügt. Durch einen auffaltbaren oder aufblasbaren Einsatz, der, speziell auf die Anwendung angepasst, in konventionelle Kulturgefäße eingesetzt wird oder ein komplettes Kulturgefäß ausbildet, wird erstmals eine Verfahrensentwicklung und -simulation unter optimierten Begasungs- und Dosierungsbedingungen ermöglicht, ebenso wie blasenfreie Begasung von Zellkulturen, spezielle aufwendige Verfahren wie Fed Batch oder Dialysefermentationen. Die Ausbeute von Vorkulturen wird erheblich gesteigert; die mit der erfindungsgemäßen Apparatur durchgeführten Prozesse sind meß- und regeltechnisch, sowie verfahrenstechnisch mit Produktionsprozessen vergleichbar.

In keinem der im Labormaßstab verwendeten Gefäße des Standes der Technik werden Einbauten gemäß der vorliegenden Erfindung verwendet, die eine effiziente, turbulente Durchmischung der Reaktionsflüssigkeit mit Wirbelstrombrechern (Schikanen) unter Ausnutzung der Kreisbewegung des Schüttlers gewährleistet und gleichzeitig die Möglichkeit für eine effektive Begasung (Ausströmrichtung des Gases nach unten, daraus folgt: die Blasen bewegen sich von unten Richtung Kreisbewegung und werden an den Wirbelstrombrechern turbulent vermischt, die Neigung zur Schaumbildung minimiert) und die Möglichkeit für Dosierstrecken, Probenahme und den Einbau von Messonden ermöglicht. Weiterhin neu ist die Technik des Aufblasens oder des Auffaltens, die es ermöglicht, die Apparatur durch enge Öffnungen in beliebige Gefäße einzubringen und an beliebige Gefäße anzupassen. Die Erfindung ermöglicht somit, mit geringsten Aufwand äußerst effiziente und beliebig optimierte Reaktionsgefäße zu verwenden, die den Stand der bisherigen Technik um Größenordnungen übertreffen.

Die vorliegende ErFindung löst die beim Stand der Technik beschriebenen upscale Probleme, da es die erfindungsgemäße Apparatur für Kulturgefäße ermöglicht, Reaktions-, mess- und regeltechnische Bedingungen analog zu Fermentern zu schaffen und so eine direkte Vergleichbarkeit der Ergebnisse der Vorversuche für die spätere Produktion gegeben ist. Dies schafft drastische Zeit- und Kostenvorteile bei der Erarbeitung von neuen Produktionsverfahren und ermöglicht hohe Ausbeutesteigerungen der Vorkulturen.

Detaillierte Beschreibung der Erfindung.

Die vorliegende Erfindung umfaßt eine Apparatur, die als aufblasbarer oder auffaltbarer Einsatz in beliebige Kulturgefäße, (z.B. in Enghalskolben), eingesetzt wird und die durch Ihren Aufbau, der durch Aufblasen oder Auffalten entsteht und der durch den Zuschnitt der Folien an beliebige Kulturgefäße angepasst wird und die die Reaktionsbedingungen im Kulturgefäß erstmalig optimal gestaltet und mit den Reaktionsbedingungen großer Reaktoren auf einfache Weise vergleichbar macht. Die Volumenzunahme von der nicht aufgeblasenen oder aufgefalteten Apparatur bis zur endgültigen Verwendungsform kann bis um das 100 fache des Volumens betragen. Das Handling und die Lagerfähigkeit der Apparatur wird so wesentlich vereinfacht.

Die vorliegende Erfindung kann, muß aber nicht, hierbei mehrere Funktionen im Kulturgefäß übernehmen. Eine Übersicht über den Aufbau gibt die beiliegende Schemazeichung 1. Wirbelstrombrecher (Schikanen):
Die Apparatur besteht aus dehnbaren oder nicht dehnbaren synthetischen oder natürlichen Polymeren und mindestens einer, bevorzugt zwei, maximal 16 im definierten Winkel, 0 Grad bis 180 Grad, bevorzugt 70 bis 110 Grad, insbesondere 90 Grad, zur vom Schüttelgerät oder Rührer erzeugten Kreisströmung, angebrachten Schikanen. Sie erzeugt eine turbulente Durchmischung der Reaktionsflüssigkeit im Bereich der Schikane(n). Zur Erhöhung der Turbulenzen können die Schikane(n) gelocht sein (Düsenwirkung) (Lochgröße 1 % bis 20% des Schikanendurchmessers, bevorzugt 2 bis 10 %, insbesondere 5%) und/oder über einen Hinterschnitt verfügen, d.h. etwas Abstand zur Gefäßwand haben (Abstand 1% bis 20% des Schikanendurchmessers, bevorzugt 2 bis 10 %, insbesondere 5%). Die Schikane(n) bestehen aus einer entsprechend der gewünschten Gefäßgeometrie geschnittenen Folie, die oben in den Aufblasschlauch übergeht (PK in). Der optimale Durchmesser der Schikane beträgt 10% bis 30%, bevorzugt 10 bis 20 %, insbesondere 15% des Durchmesser des Kulturgefäßes. Am Boden des Gefäßes wird zur besseren Fixierung der Apparatur ein ebenfalls aufblasbarer Fixierring mit einem Durchmesser von 2-16 mm, bevorzugt 4 mm, oder ein der Gefäßform angepaßtes Doppel T Fixierstück, angebracht. Dieser sorgt zusätzlich für den Druckausgleich in den einzelnen Schikanen. Zur Verbesserung der Stabilität sind mehrere Schikanen durch einen oder mehrere Stege, die ebenfalls aufblasbar sind, verbunden. Diese Stege verhindern ein Umklappen" der Schikanen bei zu hoher Anströmkraft durch die Reaktionsflüssigkeit. Zur weiteren Versteifung der Schikanen kann an der Rückseite eine Schiene in die Folie der Schikane eingeschweißt sein oder ein wiederverwendbarer Einsatz zur Fixierung der Apparatur verwendet werden. Die Wirbelstrombrecher werden vor Start der Reaktion an einer Öffnung verschlossen und an der anderen Öffnung mit Prozessgas, bevorzugt Luft, mit einem Druck von 0,1 bis 3 bar, bevorzugt 0,5 bar, aufgeblasen und dann die andere Öffnung verschlossen, um die durch den Druck erfolgte Formgebung konstant zu halten.

Weiterhin ermöglichen die Wirbelstrombrecher die bei der Kultivierung von Zellen oftmals gewünschte blasenfreie Begasung und Entgasung der Reaktionstlüssigkeit. Hierbei erfolgt die Be/Entgasung nicht mit in den Reaktionsraum eingeströmte Luft (s.u.), sondern durch Diffusion des Gases aus dem Hohlraum des Wirbelstrombrechers durch die Kunststoffolie oder definierte Teile davon, für diese Anwendung bevorzugt Silikon, in die Reaktionsflüssigkeit oder aus der Reaktionsflüssigkeit. Bei dieser Anwendung werden die Aufblas- und Auslaßöffnung nicht verschlossen, sondern durch die Aufblasöffnung kontinuierlich Gas nachgeliefert und durch einen Druckregier an der Auslaßöffnung konstant unter einem Oberdruck von 0,2 bis 3 bar, bevorzugt 0,5 bar, gehalten. Eine weitere Sonderform der blasenfreien Begasung stellt der Ersatz des Gases durch Flüssigkeiten mit sonst identischen Aufbau dar. Manche Flüssigkeiten verfügen über wesentlich höhere Gaslöslichkeiten als Wasser. Benutzt man nun eine solche Flüssigkeit als Träger für ein Gas, z.B. verfügen perfluorierte Kohlenwasserstffe über sehr hohe Sauerstofflöslichkeiten, entsteht gegenüber der Reaktionsflüssigkeit ein Diffusionsgradient, der zum Eindiffundieren des gewünschten Gases, zB. 02 oder im umgekehrten Fall zur Abdiffusion von in der Reaktionsflüssigkeit entstandenen Gasen, z.B. CO2, führt. Weiterhin ist es möglich, die Folien der Wirbelstrombrecher oder Teile davon aus Polymeren herzustellen, die für Substanzen definierter Molekülgröße durchlässig sind. Auf diese Weise kann von der Flüssigkeit aus, die die Wirbelstrombrecher auf oben genannte Weise in Form hält, ein Zu- oder Abtransport von Substanzen zur oder aus der Reaktionsflüssigkeit erfolgen. Als Beispiel sei hier die Nachfütterung von Zellkulturen mit Glucose bei gleichzeitiger Entfernung des störenden Lactats genannt.

Bei Einsatz mehrerer Apparaturen in ein Kulturgefäß ist es möglich, z.B. über die eine Apparatur eine blasenfreie Begasung durchzufahren und über eine andere Apparatur durch ein integriertes Motekularsieb Substrat nachzuliefern, wobei beide Apparaturen erfindungsgemäß die Funktion der Wirbelstrombrecher erfüllen.

Zur Erhöhung der dem Diffusionsvorgang zugänglichen Austauschfläche kann die sonst glatte Oberfläche der Apparatur durch Zotten, Fasern, Falten, Lamellen oder sonstigen, die Oberfläche erhöhenden Geometrieformen, vergrößert werden.

Wird das durch die Apparatur fließende Fluid temperiert, funktioniert die Apparatur wie ein Wärmetauscher und ermöglicht so eine exakte Temperaturregelung des Kulturgefäßes an Stelle sonst üblicher Temperiereinheiten.

### Begasungssystem:

In die Folie, aus der die Wirbelstrombrecher geformt sind, ist neben den Wirbelstrombrechern ein Kanal von 1 mm bis 5mm Stärke, bevorzugt 2mm, eingeschweißt, der oben an der Deckeldurchführung mit einem Sterilfilter zur sterilen Begasung des Systems bestückt werden kann und der unten, zum Boden des Kulturgefäßes gerichtet, in die Reaktionsflüssigkeit mündet. Die untere Öffnung ist am unteren Steg befestigt. Durch diesen Kanal wird die dritte Phase, das Gas (PI in), in den Reaktionsraum eingebracht. An Stelle der Kanalerzeugung durch Einschweißen kann ein geeigneter Schlauch ein- oder angeschweißt werden.

Probenahmesystem:

Analog zum Begasungssystem ist neben den Wirbelstrombrechern ein Kanal mit geigneten Abmessungen eingeschweißt, der am Boden des Kulturgefäßes mündet und nach der Deckeldurchführung offen ist. Durch diesen Kanal kann mit einer Einmalspritze beliebig oft ein Aliquot der Reaktionsflüssigkeit entnommen werden (Probe out).
An Stelle der Kanalerzeugung durch Einschweißen kann ein geeigneter Schlauch ein- oder angeschweißt werden.

### Abluftsystem:

Analog zum Begasungssystem ist neben den Wirbelstrombrechern ein Kanal mit geeigneten Abmessungen eingeschweißt, der im oberen Luftraum des Kulturgefäßes mündet und nach der Deckeldurchführung offen ist. Durch diesen Kanal kann Abluft aus dem Luftraum über der Reaktionsflüssigkeit entnommen werden, z.B. um die Gaswechselrate der Reaktion zu bestimmen. An Stelle der Kanalerzeugung durch Einschweißen kann ein geeigneter Schlauch ein- oder angeschweißt werden.

### Dosierstrecken:

Analog zum Begasungssystem sind am gegenüberliegenden Wirbelstrombrecher drei Kanäle mit geeigneten Abmessungen eingeschweißt, über die beliebige Flüssigkeiten, wie Substrat, Lauge, Schaumbekämpfungsmittel in die Reaktionsflüssigkeit dosiert werden können. Außerhalb der Deckeldurchführung kann ein beliebiges Dosiergerät angeschlossen werden, die Kanäle münden am oberen Steg kurz über oder unterhalb des Flüssigkeitspiegels. An Stelle der Kanalerzeugung durch Einschweißen kann ein geeigneter Schlauch ein- oder angeschweißt werden.

### Sondenarmatur:

Die Sondenarmatur ist ein Rohr, das an der Durchführung der Deckelöffnung und durch Ösen an den Stegen befestigt ist. Der Durchmesser beträgt 2 bis 25 mm, bevorzugt 8 mm. Durch die Armatur können geeignete Messonden, wie pH Sonden, p02 Sonden, Trübungsmessung, Levelsensor für Flüssigkeitsvolumen oder Schaumdetektion direkt in die Reaktionsflüssigkeit eingebracht werden. Die Abdichtung zur Armatur hin erfolgt durch O-Ringe oder durch Einschweißen der Sonden. Ist ein direkter Sondenkontakt mit der Reaktionsflüssigkeit nicht gewünscht, können die Sonden durch eine Membran von der Reaktionsflüssigkeit getrennt werden. Die Membran muß die Diffusion der zu messenden Substanzen ermöglichen, jedoch eine sterile Absperrung zur Reaktionsflüssigkeit hin darstellen. Beispielsweise wird hierduch die Messung von gelösten Sauerstoff in sterilen Reaktionsflüssigkeiten mit unsterilen p02 Sonden ermöglicht.

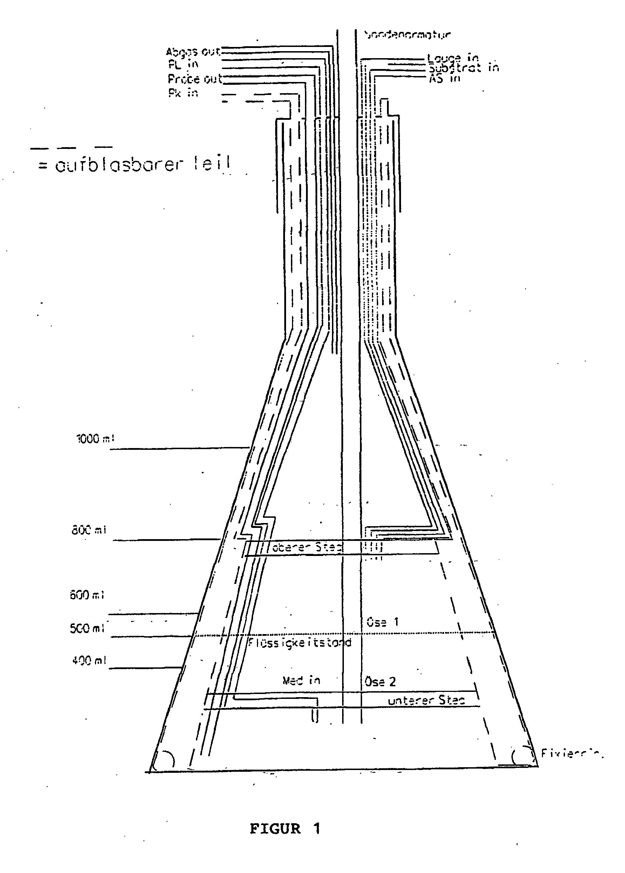

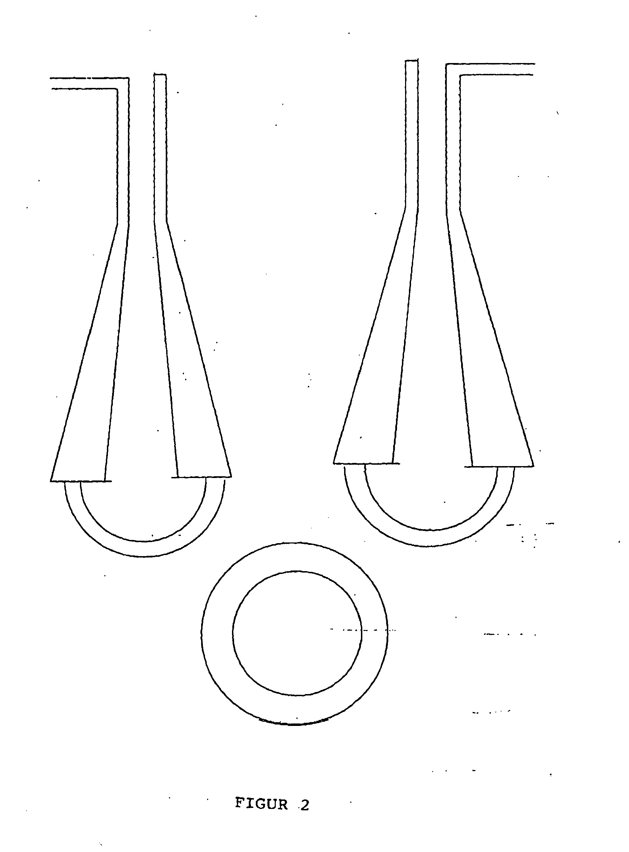

Die Beschreibung einer Ausführungsform der Erfindung erfolgt an einem 1000 ml Erlenmeyerkolben mit einem Reaktionsvolumen von 500 ml. Es wird ausdrücklich darauf hingewiesen, daß diese Beschreibung auf andere Kulturgefäße mit anderen Geometrien und Volumina (10 ml bis 5000ml) einfach übertragen werden kann. Den schematischen Aufbau zeigt die Figur 1.

Aus einer Kunststoffolie mit einer Stärke zwischen 0,1 mm bis 4 mm, bevorzugt 0,5 bis 2 mm, insbesondere 1 mm, werden zwei spiegelgleiche Teile gemäß Schnittzeichnung 2 und der zugehörige Fixierring und die beiden Stege geschnitten. Die Auswahl der Art des Kunststoffs, bevorzugt Polyurethan, und die Art des Verbindens (Heißsiegeln, Kaltsiegeln, Kleben, Schweißen, Ultraschalischweißen), bevorzugt Heißsiegeln, ist abhängig von der Vorbehandlung (z.B. Sterilisieren) und Beschaffenheit der Reaktionsflüssigkeit. Es kommen alle Kunststoffe in Frage, die bezüglich Temperaturbeständigkeit und chemischer Beständigkeit mit der Vorbehandlung und Beschaffenheit der Reaktionsflüssigkeit verträglich sind und die durch geeignete Techniken miteinander verbunden werden können.

Der Einsatz kann auch, wie die Formen von Luftballons, als aufblasbare Kunststofform, aus einem Stück bestehen. Zusätzlich ist es möglich, die Apparatur nicht nur als Einsatz für Kulturgefäße zu verwenden, sondern mit der auffaltbaren oder aufblasbaren Apparatur die Wandungen und Geometrie eines Kulturgefäßes auszubilden. Es entsteht ein aufblasbares oder auffaltbares Kulturgefäß aus Polymeren, das die erfindungsgemäßen Eigenschaften der Apparatur beinhaltet. Es lassen sich auf diese Weise Einsätze oder ganze Kulturgefäße mit Volumina von 2 ml bis 20000 m1 erfindungsgemäß herstellen.

Als Kulturgefäß wird ein 1000 ml Edenmeyerkolben mit einem Reaktionsvolumen von 500 ml benutzt. In die Abdeckkappe des Kolbens (Kappsenberg Kappe) werden zur Durchführung der Zuleitungen zur erfindungsgemäßen Apparatur entsprechende Löcher gebohrt, die Zuleitungen eingefädelt und dann mit Klebstoff dicht eingeklebt. Der Kolben wird mit der Reaktionsflüssigkeit befüllt, die Apparatur im nicht aufgeblasenen Zustand durch die Deckelöffnung in das Kulturgefäß eingeführt. Anschließend wird der Kolben mit der Kappe verschlossen. Soll die Reaktion unter sterilen Bedingungen stattfinden, wird auf die Belüftungsleitung (PL in) und die Abluftleitung (Abgas out) ein sterilisierbarer Luftfilter aufgesetzt und die anderen Zu- und Ableitungen der Apparatur, sowie die Sondenarmatur verschlossen und das gesamte Gefäß im Autoklaven sterilisiert. Anschließend wird der Kolben durch Zugabe einer Reinkultur des zu kultivierenden Mikroorganismus beimpft. Das Kulturgefäß wird in eine Schüttelapparatur eingesetzt. Erfindungsgemäß werden die Wirbelstrombrecher über die Einlassöffnung Pkin mit Luft aufgeblasen und nehmen ihre vorbestimmte Form im Kulturgefäß an. Die Ein- und Auslaßöffnungen der Wirbelstrombrecher werden anschließend verschlossen, so daß der aufgeblasene Zustand erhalten bleibt. An den Sterilfilter der Belüftungsstrecke wird eine über Durchflußmesser geregelte Begasung angeschlossen. Über das Probenahmesystem kann jetzt z.B. mittels Einmalspritze ein Aliquot der Reaktionsflüssigkeit zur externen Analytik entnommen werden. An die Abluftstrecke kann ein Analyser für die Analyse von CO2 und 02 in der Abluft angeschlossen werden. An die Dosierstrecken werden Dosierpumpen für Prozeßreagenzien, wie Lauge, Antischaummittel oder Substrat angeschlossen. In die Sondenarmatur wird eine zuvor in In Natronlauge kalt sterilisierte pH Elektrode, z.B. Typ Orion pH 9126 unter sterilen Bedingungen eingeführt. Die pH Sonde wird mit O-Ringen zur Reaktionsflüssigkeit hin abgedichtet, so daß nur das für die pH Messung nötige Diaphragma der Sonde mit der Reaktionsflüssigkeit in Verbindung steht. Die Sonde wird anschließend mit einem pH Messgerät verbunden. Anschließend wird die biologische oder biochemische Reaktion unter kulturspezifischen Bedingungen durchgeführt. Während der Kultivierung ermöglicht die erfindungsgemäße Apparatur nun die Durchführung einer Fermentation, die von den verfahrenstechnischen Gegebenheiten her mit einem hochtechnisierten Fermenter vergleichbar ist und zusätzlich die Registrierung, Regelung und Berechnung folgender Messwerte ermöglicht:

**Tabelle 1**

| Online Messwerte (d.h. kontinuierlich gemessen) | | |
|---|---|---|
| Messwert und Bezeichnung Kulturgefäß | erf. App. | Standard |
| pH Wert | ja | nein |
| 02 Gehalt im Kulturgefäß | ja | nein |
| CO2 Gehalt im Kulturgefäß | ja | nein |
| Substratdosage | ja | nein |
| Laugendosage | ja | nein |
| Antischaummitteldosage | ja | nein |
| Temperatur (über pH Sonde) | ja | nein |
| Belüftungsrate | ja | nein |

| Offline Messwerte (d.h. aus Aliquots bestimmt) | | |
|---|---|---|
| Schüttelfrequenz | ja | ja* |
| Biomasse | ja | ja* |
| Wachstumsrate | ja | ja* |
| Medienzusammensetzung | ja | ja* |
| Produktkonzentration | ja | ja* |

| Berechnete Parameter aus Online- und Offlinewerten | | |
|---|---|---|
| Sauerstoffeintragsrate | ja | nein |
| CO2 Produktionsrate | ja | nein |
| Respirationskoeffizient | ja | nein |
| Stoffübergangskoeff. KLA | ja | nein |
| Substratverbr. /Zeiteinh. | ja | nein |
| Dosagen / Zedeinheit | ja | nein |
| Produktions- und Verbrauchsraten pro Organismus | ja | nein |

| | | |
|---|---|---|
| (*) Nur möglich bei Entnahme des Kulturgefäßes aus dem Schüttler, Öffnen des Gefäßes in geeigneter Umgebung, Probenahme. D. h., die Reaktion wird für diesen Zeitraum unterbrochen. | | |

Die Berechnungsformeln für diese Werte sind in der einschlägigen Literatur hinreichend beschrieben. Insbesondere die Online gemessenen Parameter und die daraus berechenbaren Parameter sind wesentliche Voraussetzung für eine Vergleichbarkeit der Ergebnisse im Kulturgefäß mit den Ergebnissen der späteren Produktionsfermenter. Erfindungsgemäß ermöglicht der Aufbau der Apparatur nicht nur die Erfassung dieser Parameter, sondern ermöglicht erst auf Grund seines Aufbaus, diese Parameter in Größenordnungen in einem einfachen Kulturgefäß zu erreichen, wie sie später im Produktionsfermenter vorliegen werden. Der relative Stoffübergangskoeffizient KLA stellt zum Beispiel einen der wichtigsten Vergleichs- und Scale up Parameter für Reaktoren dar. Je größer der KLA Wert ist desto größer ist das Produkt aus Gasaustauschfläche A und Stoffübergangskoeffizient KL. Hält man diesen Wert in verschiedenen Reaktoren konstant, sind der Zu- und Abtransport von Gasen zur Reaküonsflüssigkeit und damit die Reaktoren vergleichbar. Voraussetzung hierfür ist, daß alle zu vergleichenden Reaktoren diese KLA Werte auch realisieren können. Für kurze und effektive Prozesse werden hohe KLA Werte gewünscht, da diese hohe Stoffwechseiraten und damit Produktausbeute ermöglichen. Vergleicht man die KLA Werte verschiedener Kulturgefäße und Reaktoren, so ergeben sich, abhängig von der Schüttelfrequenz und Belüftungsrate folgende Bereiche (relativer Vergleich)

### Aufzählung 2

| | |
|---|---|
| Erlenmeyerkolben ohne Schikanen | KLA 1% bis 3% |
| Erlenmeyerkolben mit Schikanen | KLA 1% bis 6% |
| begaste Kulturflasche | KLA 1% bis 30% |
| 1 Liter Fermenter | KLA 1% bis 800 % |
| 1 m³ Fermenter | KLA 1% bis 600 % |
| erfindungsgemäße Apparatur in Erlenmeyerkolben | KLA 1% bis 800 % |

Analoge Vergleiche werden für andere Parameter, wie Sauerstoffeintragsrate oder CO2 Abtransportrate bei Einsatz der erfindungsgemäßen Apparatur erreicht. Gegenüber dem Stand der Technik ermöglicht diese somit optimale Reaktionsbedingungen, wie sie später im Produktionsmaßstab vorliegen, und damit eine wesentliche Effektivitätssteigerung und Kostenersparnis im Bereich der Verfahrensentwicklung für biologische oder biochemische Dreiphasensysteme.

### Ausführungsbeispiel 1

Die erfindungsgemäße Apparatur wird aus Polypropylenfolien mit 0,5 mm Stärke hergestellt. Entlang der Strichlinien gemäß Schemazeichung 1 wird auf ein Schnittteil gleichmäßig Klebstoff (z.B.UHU endfest. plus 300, 2 Komponenten Epoxidharzkleber) aufgetragen und dann das andere Schnittteil exakt aufgelegt. Es ist dabei darauf zu achten, daß die Ein- und Auslaßöffnungen der Dosierkanäle nicht, und die Ränder des aufblasbaren Teils exakt und dicht verklebt werden. Nach einer Trockenzeit von 12 Stunden kann der untere Teil des Fixierrings aufgeklebt werden. Ebenso werden die beiden Stege (in diesem Schnittmuster nicht aufblasbar) eingeklebt und die Dosierkanäle für Belüftung, Dosierstrecken und die beiden Ösen für die Sondenarmatur auf die Stege aufgeklebt. Nach einer weiteren Trockenzeit von 12 Stunden ist die Apparatur einsetzbar. Die erfindungsgemäße Apparatur kann auch von spezialisierten Herstellern als ein mit Gas formbarer Kunststoffkörper aus einem Stück hergestellt werden. Als Kulturgefäß wird ein 1000 ml Erlenmeyerkolben (Enghals) mit Kappsenberg Kappe verwendet. In die Kappe werden Durchführungsöffnungen für die jeweiligen Zuführungen der Apparatur gemäß Schemazeichung 1 gebohrt, die jeweiligen Kanäle durch die Kappe gefädelt und mit Klebstoff (z.B.UHU endfest plus 300, 2 Komponenten Epoxidharzkleber) dicht eingeklebt. Nach einer Trockenzeit von 12 Stunden wird die Apparatur, nach Befüllen des Kulturgefäßes mit 500 ml Medium, im nicht aufgeblasenen Zustand, in das Kulturgefäß eingesetzt und alle Zuführungen mit Schlauchklemmen verschlossen. Nach Aufsetzen der Kappe kann das Kulturgefäß im Autoklaven sterilisiert werden. Für das Durchführen der Kanäle kann auch ein Adapter oder Kupplungsstück in die Kappe eingearbeitet werden. Als Referenzkulturgefäß wird ein Erlenmeyerkolben ohne die erfindungsgemäße Apparatur und ebenfalls ohne Schikanen (Ref.1), sowie ein Erlenmeyerkolben mit Schikanen, aber ohne die erfindungsgemäße Apparatur (Ref.2) verwendet. Beide werden ebenfalls mit 500 ml Medium befüllt, mit einer Kappe verschlossen und im Autoklaven sterilisiert.

### Mediumszusammensetzung:

| | |
|---|---|
| Hefeextrakt für die Mikrobiologie | 12 g/l |
| Glucose für die Mikrobiologie | 10 g/l |
| Ammoniumsulfat | 1,5 g/l |
| Kochsalz | 0,1 molar |
| Magnesiumchlorid | 0,5 g/l |
| Kaliumphosphatpuffer Lösungsmittel | 0,1 molar, pH 7,2 als |
| Olivenöl, extravirgine | 1 ml/l |

Die Medienbestandteile sind beim einschlägigen Fachhandel in gleicher Qualität erhältlich. Die Bestandteile Glucose und Magnesiumchlorid werden als geeignete Aliquots separat sterilisiert und anschließend unter sterilen Bedingungen zugegeben.
Nach der Sterilisation und dem Abkühlen der drei Kulturgefäße erfolgt die Beimpfung mit einer Reinkultur des Mikroorganismus mit je einem Milliliter unter sterilen Bedingungen. Die Reinkultur wurde aus einem Röhrchen mit E. coli, K12, erhältlich bei der deutschen Stammsammlung (DSM Hannover) und Kultivierung des Inhalts dieses Röhrchens in 10 ml Standard 1 Medium (Merck Darmstadt) bei 37 Grad über 12 Stunden unter sterilen Bedingungen hergestellt. Die optische Dichte der Reinkultur betrug zum Zeitpunkt der Überimpfung 1,2 OD (546 nm).

Dieses Beispiel zeigt die Überlegenheit der erfindungsgemäßen Apparatur gegenüber dem Stand der Technik in einem Dreiphasensystem mit turbulenter Durchmischung gegenüber 2 Phasensystemen zeigen. Daher wurden in diesem Beispiel die Dosierstrecken und die Abluftstrecke nicht benutzt.

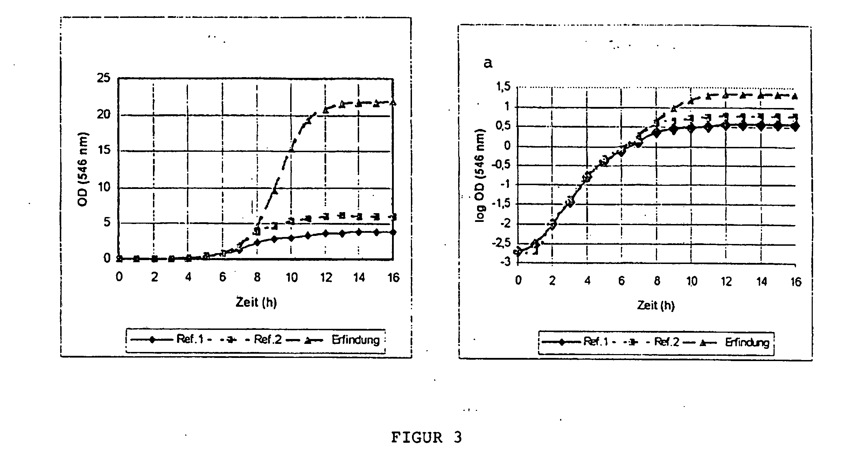

Die erfindungsgemäße Apparatur wurde mit Luft, 0,5 bar, über den Anschluß Pkin aufgeblasen, bis die endgültige Form erreicht war, und der Anschluß mit einer Schlauchklemme verschlossen, um den Druck und damit die Form zu erhalten. Alle drei Kulturgefäße wurden in einen Schüttler (B.Braun CERTOMAT BS-T) deponiert, die erfindungsgemäße Apparatur am Anschluß PLin mit einer Begasungseinheit (Vordruck 0,5 bar, Luftstrom 5 Liter/h) gekoppelt und kontinuierlich begast. Die drei Kulturgefäße wurden für 16 Stunden bei 37 Grad Celsius und einer Schüttelfrequenz von 250 RPM kultiviert. Zur Bestimmung des Wachstums der Kultur (und damit der Effektivität des Systems) wurde stündlich ein Aliquot der Kultur entnommen und die optische Dichte (OD) bei 546 nm in einem Photometer bestimmt. "Ab OD = 1,0 sind entsprechende Verdünnungen durchzuführen, um Unlinearitäten des Photometers zu kompensieren.
Tabelle 2 und Figur 3 zeigen deutlich das bessere Wachstum der kultivierten Mikroorganismen in einem erfindungsgemäßen Bioreaktor. In der Fig. 3 sind die OD Werte logarithmisch aufgetragen (siehe auch Tab. 2). Mit der Erfindung wird gegenüber Ref.1 ein 5,7-fach besserer und gegenüber Ref.2 ein 3,7-fach besserer OD-Wert erhalten. Da die optische Dichte direkt die Wachstumsrate des Organismus und damit indirekt seine Stoffwechselrate wiedergibt, zeigt sie als meßbarer Parameter deutlich die mit der Erfindung erreichte Effektivitätssteigerung.

### Ausführungsbeispiel 2

Die Herstellung der erfindungsgemäßen Apparatur erfolgt analog dem Ausführungsbeispiel 1. Als Kulturgefäß wird ein 1000 ml Erlenmeyerkolben (Enghals) mit Kappsenberg Kappe verwendet. In die Kappe werden Durchführungsöffnungen für die jeweiligen Zuführungen der Apparatur gemäß Schemazeichung 1 gebohrt, die jeweiligen Kanäle durch die Kappe gefädelt und mit Klebstoff (UHU endfest plus 300, 2 Komponenten Epoxidharzkleber) dicht eingeklebt. Nach einer Trockenzeit von 12 Stunden wird die Apparatur, nach Befüllen des Kulturgefäßes mit 450 ml Medium, im nicht aufgeblasenen Zustand, in das Kulturgefäß eingesetzt und alle Zuführungen mit Schlauchklemmen verschlossen. Nach Aufsetzen der Kappe und Einführung einer sterilisierbaren, geeichten pH Elektrode in die Sondenarmatur kann das Kulturgefäß im Autoklaven sterilisiert werden. Als Referenz (Ref.3) wird eine Minifermenter Typ LAB-FORS der Firma lnfors verwendet. Dieser repräsentiert ebenfalls ein Dreiphasensystem mit eingebauten Schikanen, wird aber nicht geschüttelt, sondern ist mit einem Rührwerk versehen. Er stellt im Gegensatz zu den in Ausführungsbeispiel 1 beschriebenen Kulturgefäßen die kleinst mögliche Einheit eines Fermenters dar. Die mit dieser Anlage erhaltenen Daten können für ein Scaie up des Prozesses in den Produktionsmaßstab verwendet werden. Mit diesem Ausführungsbeispiel soll gezeigt werden, daß die erfindungsmäße Apparatur bei einem Einsatz in einfachen Kulturgefäßen in der Lage ist, die Leistungsdaten dieser hochtechnisierten Fermenteranlage zu erreichen und zu übertreffen. Das Fermentationsgefäß wird mit 450 ml Medium befüllt und nach Herstellervorschrift im Autoklaven sterilisiert.

### Mediumszusammensetzung:

| | |
|---|---|
| Hefeextrakt für die Mikrobiologie | 20 g/l |
| Ammoniumsulfat | 1, 5 g/l |
| Kochsalz | 0,1 molar |
| Magnesiumchlorid | 0,5 g/l |
| Kaliumphosphatpuffer Lösungsmittel | 0,1 molar, pH 7,2 als |
| Antischaummittel | 1 ml/l |
| (Silikonöl Dow Corning) | |

Die Medienbestandteile sind beim einschlägigen Fachhandel in gleicher Qualität erhältlich. Der Bestandteil Magnesiumchlorid wird als geeignetes Aliquot separat sterilisiert und anschließend unter stehlen Bedingungen zugegeben. Als Substratvorlage dient Glucose (20g in 50ml), als Laugenvorlage dient 25%ige Ammoniaklösung (Ammoniakzugabe unter sterile Bedingungen nach der Sterilisation des Vorlagegefäßes), Inhalt 50 ml und als Antischaumvorlage dient 10% Silikonöl (Dow Corning), Inhalt 50 ml.

Nach der Sterilisation und dem Abkühlen der zwei Kulturgefäße erfolgt die Beimpfung mit einer Reinkultur des Mikroorganismus mit je einem Milliliter unter sterilen Bedingungen. Die Reinkultur wurde aus einem Röhrchen mit E. coli, K12, erhältlich bei der deutschen Stammsammlung (DSM Hannover) und Kultivierung des Inhalts dieses Röhrchens in 10 ml Standard 1 Medium (Merck Darmstadt) bei 37 Grad über 12 Stunden unter sterilen Bedingungen hergestellt. Die optische Dichte der Reinkultur betrug zum Zeitpunkt der Überimpfung 1,36 OD (546 nm). Die erfindungsmäße Apparatur wird wie in Ausführungsbeispiel 1 beschrieben, vorbereitet und in einen Schüttler (B. Braun CERTOMAT BS-T) deponiert, am Anschluß PLin mit einer Begasungseinheit gekoppelt (Vordruck 1 bar, Luftstrom 15 1/h) gekoppelt und kontinuierlich begast. Die pH Elektrode wird mit einem pH Mess-und Regelgerät verbunden. An die Dosierstrecken werden die Substratvorlage, die Laugenvorlage und die Antischaumvorlage angeschlossen. An die Abluftleitung Abgas out wird ein Messgerät zur Bestimmung des 02 und CO2 Gehaltes der Abluft angeschlossen, um den KLA Wert des Systems berechnen zu können. Die Schüttelfrequenz für diesen Versuch beträgt 350 RPM. Das Gefäß wird auf 37 Grad Celsius temperiert. Der Referenzfermenter Ref.3 wird gemäß Herstellervorschrift mit der pH Mess- und Regeltechnik, der Substratdosage, der Laugendosage und Antischaumdosage verbunden und an der Abgasstrecke ebenfalls an eine 02 und CO2 Messung angeschlossen. Die Drehzahl des Rührwerks beträgt 600 RPM, der Luftstrom zur Belüftung ebenfalls 151/h. Der Fermenter wird auf 37 Grad Celsius temperiert.

### Dosageparameter für beide Ansätze

| | |
|---|---|
| Substratdosage | kontinuierlich mit 2,5 ml /h (1g Glucose pro h) |
| Laugendosage | zur Regelung des pH Wertes, Soll pH > 7,0 |
| Antischaumdosage | nur bei Bedarf, d.h. Überschäumen der Kultur |

Beide Ansätze wurden jeweils für 20 Stunden kultiviert. Zur Bestimmung des Wachstums der Kultur (und damit der Effektivität des Systems) wurde stündlich ein Aliquot der Kultur entnommen und die optische Dichte (OD) bei 546 nm in einem Photometer bestimmt. Ab OD = 1,0 sind entsprechende Verdünnungen durchzufahren, um Unlinearitäten des Photometers zu kompensieren. Die KLA Werte wurden nach der dynamischen Methode «Bandyopadhyay,B, Humphrey,A, and Taguchi, H, 1967, Biotechnoi. and Bioeng. 9, 533) berechnet.

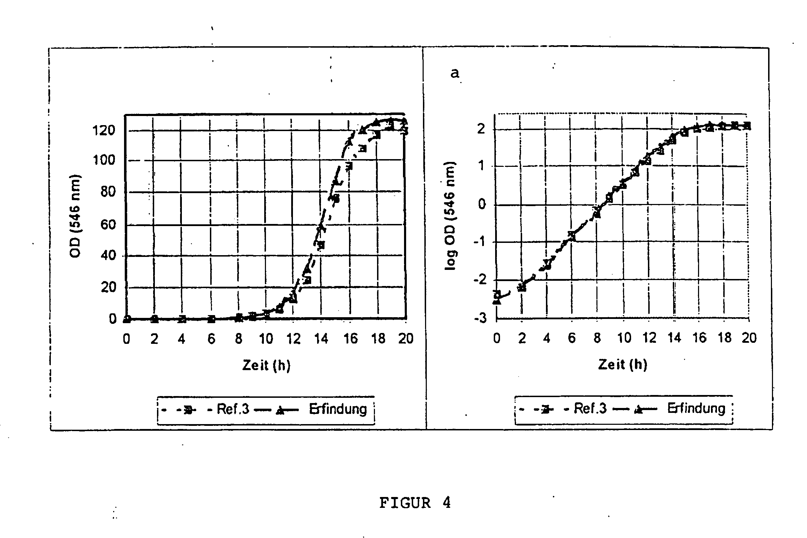

Tabelle 3 und Figur 4 zeigen deutlich die Vergleichbarkeit, bzw. die Überlegenheit der erfindungsgemäßen Apparatur gegenüber dem Stand der Technik bei Fermentern. In Tabelle 3a und Figur 4a sind die OD Werte logadthmisch aufgetragen. Wie im Ausführungsbeispiel 1 wird die optische Dichte als Maß für die Effektivität des Reaktors verwendet. Ein Vergleich der dynamisch bestimmten KLA Werte zeigt gut die Vergleichbarkeit der verfahrenstechnischen und biologischen Bedingungen zwischen beiden Ansätzen.

| | |
|---|---|
| KLA Wert Referenzkultur Ref.3 | 210 ± 24 (1/h) |
| KLA Wert Erfindung | 218 ± 27 (1/h) |

Die Wachstumskurven und absoluten OD Werte sind praktisch identisch (bei der Erfindung geringfügig höher), was eine ausgezeichnete Vergleichbarkeit der beiden Systeme beweist, und somit zeigt, daß die erfindungsgemäße Apparatur herkömmlichen, bisher verwendeten Kulturgefäßen weit überlegen und mit hochwertigen Fermentationsanlagen mindestens vergleichbar ist.

Die Erfindung kann mit den folgenden Merkmalen kombiniert sein, nämlich mit einem Verfahren und einer Vorrichtung zur Erzeugung eines Carrierfluids, das gleichzeitig zur Begasung des Kulturgefäßes verwendet werden kann. Dem Carrierfluid

| Tabelle 2 OD der drei Kulturgefäße | | | | | Tabelle 2a log OD der drei Kulturgefäße | | | |
|---|---|---|---|---|---|---|---|---|
| Zeit (h) | Ref. 1 | Ref. 2 | Erfindung | | Zeit (h) | Ref.1 | Ref.2 | Erfindung |
| 0 | 0,002 | 0,002 | 0,002 | | 0 | -2,698970004 | -2,698970004 | -2,698970004 |
| 1 | 0,003 | 0,002 | 0,003 | | 1 | - 2,522878745 | - 2,698970004 | - 2,522878745 |
| 2 | 0,01 | 0,009 | 0,01 | | 2 | -2 | - 2,045757491 | -2 |
| 3 | 0,038 | 0,042 | 0,04 | | 3 | - 1,420216403 | - 1,37675071 | - 1,39794000 9 |
| 4 | 0,16 | 0,146 | 0,15 | | 4 | 0,795880017 | -0,835647144 | - 0,823908741 |
| 5 | 0,41 | 0,48 | 0,36 | | 5 | 0,387216143 | -0,318758763 | -0,443697499 |
| 6 | 0,76 | 0,87 | 0,86 | | 6 | 0,119186408 | -0,060480747 | -0,065501549 |
| 7 | 1,31 | 1,53 | 1,89 | | 7 | 0,117271296 | 0,184691431 | 0,276461804 |
| 8 | 2,37 | 3,87 | 4,56 | | 8 | 0,374748346 | 0,587710965 | 0,658964843 |
| 9 | 2,82 | 4,65 | 9,65 | | 9 | 0,450249108 | 0,667452953 | 0,984527313 |
| 10 | 3,12 | 5,32 | 15,45 | | 10 | 0,494154594 | 0,725911632 | 1,188928484 |
| 11 | 3,34 | 5,56 | 19,46 | | 11 | 0,523746467 | 0,745074792 | 1,289142836 |
| 12 | 3,67 | 5,87 | 20,8 | | 12 | 0,564666064 | 0,768638101 | 1,318063335 |
| 13 | 3,72 | 6,01 | 21,6 | | 13 | 0,57054294 | 0,778874472 | 1,334453751 |
| 14 | 3,85 | 5,98 | 21,86 | | 14 | 0,58546073 | 0,776701184 | 1,339650158 |
| 15 | 3,82 | 5,94 | 21,76 | | 15 | 0,582063363 | 0,773786445 | 1,337658891 |
| 16 | 3,84 | 5,97 | 21,89 | | 16 | 0,584331224 | 0,775974331 | 1,340245762 |

| Tabelle 3 OD der beiden Kulturen | | | | | Tabelle 3a log OD der beiden Kulturen | |
|---|---|---|---|---|---|---|
| Zeit (h) | Ref.3 | Erfindung | | Zeit (h) | Ref.3 | Erfindung |
| 0 | 0,004 | 0,003 | | 0 | - 2,39794000 9 | -2,52287874 5 |
| 2 | 0,006 | 0,007 | | 2 | - 2,22184875 | - 2;15490196 |
| 4 | 0,026 | 0,023 | | 4 | - 1,58502665 2 | - 1,63827216 4 |
| 6 | 0,15 | 0,14 | | 6 | - 0,82390874 1 | - 0,85387196 4 |
| 8 | 0,67 | 0,56 | | 8 | -0,17392519 7 | - 0,25181197 3 |
| 9 | 1,38 | 1,58 | | 9 | 0,139879086 | 0,198657087 |
| 10 | 2.98 | 3,45 | | 10 | 0,474216264 | 0,537819095 |
| 11 | 6,12 | 7,23 | | 11 | 0,786751422 | 0,859138297 |
| 12 | 12,56 | 16,41 | | 12 | 1,098989639 | 1,215108581 |
| 13 | 24,1 | 31,8 | | 13 | 1,382017043 | 1,50242712 |
| 14 | 46,2 | 58,7 | | 14 | 1,664641976 | 1,768638101 |
| 15 | 75,8 | 86,9 | | 15 | 1,879669206 | 1,939019776 |
| 16 | 96,4 | 112,8 | | 16 | 1,984077034 | 2,0523091 |
| 17 | 107,8 | 119,8 | | 17 | 2,032618761 | 2,078456818 |
| 18 | 116,7 | 124,7 | | 18 | 2,067070856 | 2,095866453 |
| 19 | 121,5 | 126,8 | | 19 | 2,084576278 | 2,103119254 |
| 20 | 118,9 | 126,1 | | 20 | 2,075181855 | 2,100715087 |

können quantitativ und definiert die zu dosierenden Fluide beigemischt werden. Ohne Verwendung von Pumpen und anderen aufwendigen mechanischen Teilen können so im Kulturgefäß in der Reaktionsflüssigkeit und in der Atmosphäre des Gefäßes definierte und geregelte Bedingungen geschaffen werden, wobei gleichzeitig die Eigenschaften der dosierten Fluide optimal genutzt werden. Besonders geeignet ist die Erfindung für den parallelen Betrieb mehrerer Kulturgefäße. Die vorliegende Erfindung ist angewendbar in allen Bereichen, in denen in Kulturgefäßen biologische oder biochemische Reaktionen durchgeführt werden, speziell im Bereich Biotechnologie, Lebensmitteltechnik und Umweltschutz.

Das Verfahren ist dadurch gekennzeichnet, daß das zu dosierende Fluid oder die zu dosierenden Fluide einem oder mehreren Träger- und Transportfluiden (Carrierfluiden) in definierter Konzentration beigemischt werden und dieses Carrierfluid, bzw. diese Carrierfluide dem Kulturgefäß in definierter Menge und/oder Zeiteinheiten entwender ins Reaktionsmedium oder in den Headspace zugeführt werden.

Die Vorrichtung ist gekennzeichnet durch Vorrichtungen zur Beimischung von einem oder mehreren zu dosierenden Fluiden zu einem oder mehreren Carrierfluiden und die Zuführung in ein oder mehrere Kulturgefäße, wie Sie in den folgenden Beispielen und den Patentansprüchen beschrieben werden.

Folgend erfolgt die Beschreibung beispielhaft mit der Beschreibung der einzelnen Module und erfindungsgemäßen Eigenschaften an einem 1000 ml Kulturgefäß mit 500 ml Flüssigkeitsvolumen. Es wird ausdrücklich darauf hingewiesen, daß die Zahlen (vor allem die relativen Angaben) an Kulturgefäße mit Volumen von 1 ml bis 50 m³ angepasst werden können, wobei die Querschnitte der Düsen und Dosierstrecken jeweils anzupassen sind.

### a) Gas als Transportmedium der Vorrichtung

Das Modul Gasversorgung der Vorrichtung besteht aus folgenden, wesentlichen Bauteilen (Zeichnung 1):
- Druckgaseinlaß
- Dreiwegeventil DV1 oder Einlaß- und Auslaßventil
- Gasbehälter B1
- Gasfilter F 1
- Druckausgleichskanal DG1

Der Druckgaseinlaß mit einem Eingangsüberdruck gegenüber dem Kulturgefäß von 0,1 bis 10 bar, bevorzugt 0,2 bis 1 bar, insbesondere 0,5 bar, ist über einen druckbeständigen Schlauch, Innendurchmesser 0,5 bis 8 mm, bevorzugt 0,5 bis 2mm, insbesondere 1 mm, mit dem Dreiwegeventil DV1 (s. Zeichnung 1) verbunden. Das Ventil DV1 ist so geschaltet, daß der Gasbehälter B1 mit einem Behältervolumen von 1% bis 40%, bevorzugt 1 bis 10%, insbesondere 5%, des Flüssigvolumens im Kulturgefäß, mit Druckluft oder einem anderen Gas gefüllt wird). Das Füllvolumen des Gasbehälters kann über einen eingebauten Stempel von 0% bis 1 00% des Behältervolumens variiert werden. Nach Erreichung des Druckausgleiches wird das Ventil DV1 in die andere Stellung, Gasbehälter - Kulturgefäß, geschaltet. Es entsteht durch den Druckausgleich zum Kulturgefäß hin ein Gasstrom, der nach einem optionalen Gasfilter durch nachstehend beschriebene Module geleitet werden kann und letztlich im Headspace oder der Reaktionsflüssigkeit des Kul turgefäßes ausströmt. Die nach dem Dreiwegeventil DV1 abzweigende Druckausgleichskapillare sorgt für einen ausgeglichenen Druck zwischen der Gaszuführung und den Modulen Flüssigkeitsvorlage. Am Ausgang des Moduls Gasversorgung kann ein Filter zur Filtration des Transportmediums angebracht sein. Das Kulturgefäß wird durch diese erfindungsgemäße Vorrichtung diskontiunierlich auf einfachste Weise mit definierten und damit quantifizierbaren "Gasportionen" versorgt. Je kleiner das Behältervolumen und je höher die Taktrate des Ventils ist, desto mehr nähert sich dieser diskontinuierliche Gasstrom einem kontinuierlichen Gasstrom an. In folgender Tabelle ist das Behältervolumen 5% des Flüssigvolumens der Reaktionsflüssigkeit (Beispiel 25 ml Behältervolumen, 500 ml Reaktionsflüssigkeitsvolumen) und die Begasungsrate VF der Quotient aus Gasvolumen/h zu Volumen Reaktionsflüssigkeit.

**Tabelle 1**

| Taktrate Ventil/min | Gasstrom/min in % Flüssigvolumen | VF (l /h) |
|---|---|---|
| 0 | 0 | 0 |
| 1 | 5% | 3 |
| 2 | 10% | 6 |
| 5 | 25% | 15 |
| 10 | 50% | 30 |
| 15 | 75% | 45 |
| 20 | 100% | 60 |
| 25 | 125% | 75 |
| 50 | 250% | 150 |

Für aerobe, biologische oder (bio)chemische Reaktionen liegen die VF Werte üblicherweise zwischen 5 und 60 (l/h).
Dies lässt sich mit vorliegenden, erfindungsgemäßen Modul auf einfachste Weise in einem nahezu "kontinuierlichen" Gasstrom erreichen, wobei auf komplizierte, mechanische oder elektronische Durchflußmessungen und -Regler verzichtet werden kann. Wesentlich für eine optimale und kontiniuierliche Gasversorgung von Kulturen von Mikroorganismen bei optimaler Ausnutzung des Gases ist der sogenannte "gas hold up", also die Aufenthaltszeit der Gasblasen in der Reaktionslösung, während der an der Grenzfläche zwischen Gasblase und Flüssigkeit durch Diffusion der Gasaustausch erfolgen kann. Ein Optimum der Nutzung des Gases bei gleichzeitig optimaler Begasungsrate erreicht man, wenn der "gas hold up" gleich der Taktrate des Ventils DV1 ist. Es erfolgt immer eine Dosierung von Gas, wenn die Gasblasen aus der Flüssigkeit verschwinden. Eine Variation der Menge des durchgesetzten Gases kann über das variable Volumen des Gasbehälters erfolgen. Weiterhin reduziert der erfindungsgemäße Aufbau des Moduls die Tendenz zur Schaumbildung, da immer nur soviel Gas dosiert wird, wie für eine optimale Versorgung der Kultur nötig ist.

### b) Flüssigkeit als Transportmedium

An Stelle des Moduls Gasversorgung kann Flüssigkeit als Transportmedium eingesetzt werden. In diesem Fall wird das Modul Gasversorgung durch eine geregelte Flüssigkeitspumpe ersetzt, die entweder mit einer Saugleitung mit der Reaktionsflüssigkeit im Kulturgefäß verbunden ist und diese umwälzt oder aus einem eigenen Vorratsgefäß ansaugt (Zeichnung 2). Das Modul Antriebspumpe besteht aus folgenden wesentliche Bauteilen:
- Flüssigkeitspumpe
- Saugleitung
- Druckleitung zum Kulturgefäß hin
- Filter (optional)

Der Einsatz von Flüssigkeit als Transportmedium bietet sich besonders dann an, wenn die Raktionsflüssigkeit effektiv, aber unter Vermeidung von Gasblasen in der Kultur, mit Gasen angereichert werden soll, z.B. CO2 Dosierung in Zellkulturmedien oder minimale Substanzmengen zudosiert werden sollen. Hier ist zum Beispiel die Dosage von Katalysatoren oder die Dosage von biologischen Wirkstoffen zu nennen. Wirkstoffe sind meist extrem teuer und nur in konzentrierter Form langzeitstabil. Sie werden erfindungsgemäß mit Fiüssigkeitsmodulen (s.u.) in kleinsten Mengen und in beliebiger Kombination dosiert.

### c) Modul Flüssigkeitsvorlage

Das Modul Flüssigkeitsvodage besteht aus folgenden wesentlichen Bauteilen (Zeichnung 1):
- Vorratsbehälter Flüssigkeit
- Druckausgleichsleitung, abgezweigt aus der Druckausgleichskapillare
- Zuleitung zum Taktventil und zur Venturidüse
- Taktventil
- Venturidüse

Die Flüssigkeitsvorlage ist mit einer zur Reaktionsflüssigkeit im Kulturgefäß zu dosierenden Flüssigkeit gefüllt, wobei ein Restvolumen von Gas von mindestens 2% des Volumens der Vorlage zum Druckausgleich vorhanden sein muß. Ist das Transportmedium eine Flüssigkeit, entfällt das Restvolumen das Gases in der Vorlage und der Druckausgleich über Kapillaren (Zeichnung 2). Dafür kann die Vorlage zur Vermeidung von Unterdruck mit atmosphärischem Außendruck belüftet werden. Die Flüssigkeitsvorlage kann beliebig, hängend, stehend, liegend zur Vorrichtung angebracht werden, wobei die Druckausgleichsleitung in das vorhandene Gasvolumen münden sollte. Die Flüssigkeitsvorlage hat gegenüber dem Flüssigkeitsvolumen der Reaktionsflüssigkeit ein Volumen von 0,5% bis 50%, bevorzugt 5%. Sie ist über eine Zuleitung mit dem Taktventil V1, dieses mit der Venturidüse VD1, verbunden. Liefert das Modul Gasversorgung oder Antriebspumpe einen Strom von Transportmedium über die Venturidüse, entsteht am Seiteneinlass der Düse ein Unterdruck gegenüber dem sonst druckausgeglichenen System. Bei gleichzeitiger Öffnung des Taktventils V1 wird somit Flüssigkeit aus der Flüssigkeitsvodage zum Gasstrom in der Düse hin angesaugt. Die angesaugte Menge der Flüssigkeit korreliert mit folgenden Parametern

### Tabelle 2

- Düsenabmessungen
- Druck und Gasström über die Düse
- Querschnifte der Zuleitung und des Taktventils
- Viskosität der Flüssigkeit
- Temparatur
und kann daher-quantitativ und reproduzierbar kalibriert werden. Es läßt sich folglich nur durch die Taktzeit des Ventils V1 bei konstanten Parametern gemäß Tabelle 2 eine quantitative Dosierung von Flüssigkeitsaliquots zum Transportmedium durchfuhren. Im Auslaß der Düse wird die angesaugte Flüssigkeit und das Transportmedium homogen vermischt. Zwischen dem Modul Gasversorgung oder Modul Antrieb (Zeichnung 5 und 6) und dem Modul Kulturgefäß können mehrere Module Flüssigkeitsvorlage, bevorzugt 4 Module, zwischengeschaltet werden. Die Schaltung kann parallel (bevorzugt) oder hintereinander erfolgen. Auf diese Weise wird es möglich, in das Transportmedium gleichzeitig keine bis mehrere unterschiedliche Flüssigkeiten quantitativ zu dosieren, diese in beliebiger Menge zu kombinieren und vor dem Einlaß ins Kulturgefäß homogen zu durchmischen. In biologischen Kulturen sind neben der Titration des pH Wertes mit Säuren und Laugen und dem Zusatz von Mitteln zur Schaumbekämpfung vor allem sogenannte "fed batch" Verfahren gebräuchlich. Hierbei wird der Kultur geregelt ein oder mehrere Substrate, z.B. Kohlenstoff oder Stickstoffquelle, zudosiert. Die vorliegende Vorrichtung ermöglicht auf einfachste Weise, die Zusammensetzung der Flüssigdosage zu variieren. So können zeitabhängig oder in Abhängigkeit von kulturspezifischen Regelparametern nur über die Variation der Taktzeit Substratgradienten erstellt werden oder zusätzliche Nährstoffe beigemischt werden, z.B. Wachstumsfaktoren, Mineralien oder Vitamine aus weiteren Flüssigkeitsmodulen.

### d) Modul Dosiervorlage für Gase

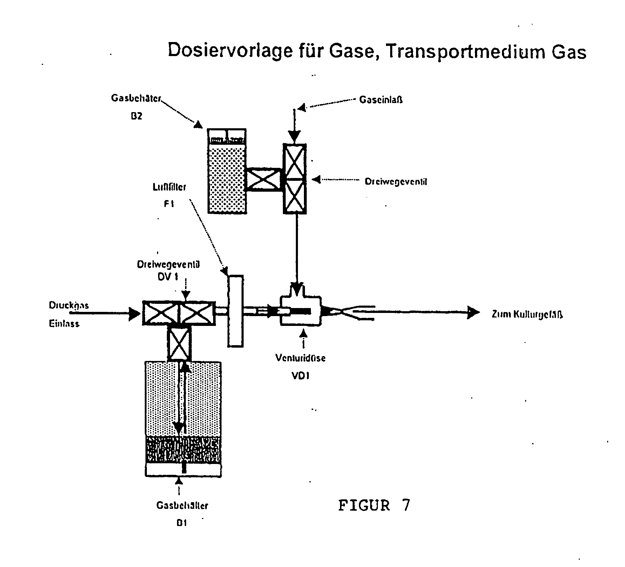

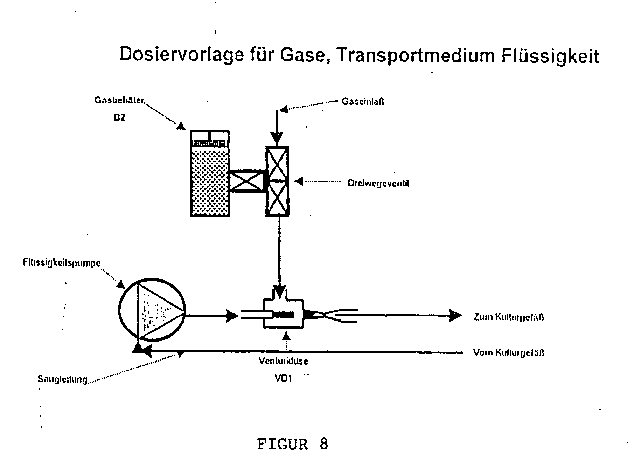

Das Modul Dosiervorlage für Gase (Figuren 7 und 8) besteht aus folgenden wesentlichen Bauteilen
- Gasbehälter B2, über Stempel im Füllvolumen verstellbar
- Gaseinlaß
- Dreiwegeventil

Das Dreiwegeventil wird vom Gaseinlaß zum Gasbehälter B2 geschaltet. Der Behälter füllt sich mit Gas, wobei über den eingebauten Stempel das Füllvolumen variiert werden kann, somit aber quantitativ bekannt ist. Soll nun eine Gasdosage erfolgen, wird das Dreiwegeventil für eine definierte Taktzeit zur Venturidüse hin umgeschaltet, wobei sichergestellt sein sollte, daß an der Düse ein Unterdruck, der durch das Transportmedium erzeugt wird, anliegt. Bei bekanntem Vordruck am Gaseinlaß, Füllvolumen des Gasbehälters und der Taktzeit des Dreiwegeventils läßt sich so eine quantitative Gasdosage erreichen. Zwischen dem Modul Gasversorgung oder Modul Antrieb (Zeichnung 1 und 2) und dem Modul Kulturgefäß können mehrere Module Gasdosage, bevorzugt 2 Module, zwischengeschaltet werden. Die Schaltung kann parallel (bevorzugt) oder hintereinander erfolgen. Auf diese Weise wird es möglich, in das Transportmedium gleichzeitig kein bis mehrere unterschiedliche Gase quantitativ zu dosieren, diese in beliebiger Menge zu kombinieren und vor dem Einlaß ins Kulturgefäß homogen zu durchmischen. Die Gasmodule können an Stelle oder in beliebiger Kombination mit den Flussigkeitsmodulen eingesetzt werden. In biologischen Zellkulturen wird haufig CO2 zur Regelung des pH Wertes eingesetzt, das mit diesem Modul unter Vermeidung von Gasblasen in der Reaktionsflüssigkeit einfach und quantitativ dosiert werden kann. Weiterhin kann durch die Gasdosage eine künstliche Atmosphäre im Kulturgefäß geschaffen und geregelt werden, die für biologische Kulturen vorteilhaft ist. Zu nennen sind hier beispielsweise die Kultur von Pflanzenzellen, die eine höhere CO2 Konzentration (als Substrat) bevorzugen oder die Anzucht anaerober Organsimen unter Stickstoff- oder Schwefelatmosphäre .

### e) Modul Kulturgefäß

Das Modul Kulturgefäß besteht im wesentlichen aus folgenden Bauteilen:
- Kulturgefäß KG1, gefüllt mit der Reaktionsflüssigkeit und darüber liegenden Gasraum (headspace) und Verschluß des Gefäßes
- Zuleitung für das Transportmedium
- Einlaßventil EV1 mit Zuleitung in den Headspace des Kulturgefäßes
- Einlaßventil EV2 mit Zuleitung in die Reaktionsflüssigkeit
- Belüfterdüse BDI in der Reaktionsflüssigkeit
- Ausströmerdüse AD1 im Headspace des Kulturgefäßes

Durch die am Verschluß des Kulturgefäßes angebrachten Einlaßventile kann gewählt werden, ob das Transportmedium in den Luftraum des Kulturgefäßes (headspace) oder in die Reaktionsflüssigkeit dosiert werden soll. Das Einlassventil EV1 zum headspace führt zu einer im Luftraum angebrachten Verneblerdüse AD1, die nochmals eine Zerstäubung des Transportmediums bewirkt. Das gesamte, vernebelte Transportmedium und die Dosagen sinken einheitlich auf die Oberfläche der Reaktionsflüssigkeit. Diese Feinverteilung bewirkt eine schnelle Mischung des Transportmediums und der Dosagen mit der Reaktionsflüssigkeit und kann zu einer effektiveren Nutzung der dosierten Flüssigkeit führen. Die Effektivität von Antischaummitteln, die in dieser Weise dosiert werden, kann hierdurch bis um das Zehnfache gesteigert werden, folglich der Verbrauch entsprechend minimiert werden. Weiterhin ist es möglich, nur einen Gasstrom ohne zudosierte Flüssigkeit zur Schaumbekämpfung einzusetzen. Der Schaum wird durch den Strom des Gases einfach "niedergeblasen". Häufig reicht dieser Effekt für die Schaumbekämpfung schon aus, ohne daß nachfolgend Antischaummittel in oben beschriebener Weise dosiert werden muß. Die Vermeidung von Antischaummitteln in biologischen Prozessen ist oberstes Ziel, da diese negative Auswirkungen auf die Kultur selbst und den späteren Aufreinigungsprozess haben können, sowie biologisch schlecht abbaubar sind und somit nicht einfach entsorgt werden können. Headspace Dosierungen in oben beschriebener Weise werden überwiegend eingesetzt werden, wenn nur eine Oberflächenbelüftung der Reaktionsflüssigkeit gewünscht ist, z.B. bei anaeroben Kulturen oder wenn Flüssigkeiten dosiert werden, die eine schneite Wirkung auf die Reaktionsflüssigkeit haben sollen. Als Beispiel sei hier die Titration des pH Wertes mit Säuren oder Laugen genannt, sowie die Schaumbekämpfung in oben beschriebener Weise. Das Einlaßventil EV2 führt zu einer in der Reaktionsflüssigkeit angebrachten Venturidüse BD1. Das Transportmedium (und die Dosagen) strömt durch die Belüfterdüse BD1 in die Reaktionsflüssigkeit. Hierbei wird am seitlichen Einlaß der Düse durch den entstehenden Unterdruck Reaktionsflüssigkeit angesaugt, die im Auslaßbereich der Düse effektiv vermischt wird. Sollen die Mikroorganismen (z.B. Gewebezellen) nicht den Scherkräften in der Düse ausgesetzt werden, ist die seitliche Einlaßöffnung mit einer Filtermembran verschließbar. Neben dem Mischeffekt, der die Mischzeiten der Reaktionsflüssigkeit drastisch verkürzt undden deutlich beschleunigten Gasaustauschraten entstehen (bei Transportmedium Gas) sehr viel kleinere Luftblasen als bei Begasungen entsprechend dem Stand der Technik. Diese kleineren Blasen erhöhen die für den Gasaustausch zwischen Luftblase und Reaktionsflüssigkeit zur Verfügung stehende Grenzfläche, erhöhen also die Gasaustauschrate und verbleiben länger in der Reaktionsflüssigkeit als große Blasen, erhöhen also den "gas hold up" und somit wieder die Gasaustauschrate. Das Gas wird effektiver genutzt, so daß, abhängig von der Art der Kultivierung, auf ein Schütteln oder Rühren des Kulturgefäßes ggf. verzichtet werden kann. Desweiteren wird durch kleinere Blasen die Tendenz zur Schaumbildung minimiert. Werden auf diese Weise Aerosole dosiert, z.B. Substrate im Gasstrom, führen die kürzeren Mischzeiten zu einer schnelleren, homogenen Verteilung in der Reaktionsflüssigkeit. Substratgradienten auf Grund schlechter Mischung können vermieden werden, die Kultur wird gleichmäßig in der gewünschten Weise versorgt.

Diese Ausbildung zeichnet sich dadurch aus, das sie für einen völlig neuen Anwendungsbereich Funktionsmodule in geeigneter Weise zusammenbringt und so eine bisher aufwendige und komplexe Technik in einer einfachen, kompakten Vorrichtung vereint. Der Einsatz der Vorrichtung für biotechnologische Prozesse unter sterilen Bedingungen wird möglich. Hierdurch werden regelungstechnisch Bereiche zugänglich, die bisher mit dem Stand der Technik nicht bedient werden konnten. Als Beispiel sei hier die neuerdings durchgeführte Parallelfermentation von Kulturgefäßen, üblicherweise bis zu 16 Gefäße, genannt (Das GIP GmbH, www.dasgip.de), die der Medien und Verfahrensoptimierung von biologischen Verfahren dient. Hierbei sollen unter möglichst produktionsnahen Bedingungen Einwirkungen verschiedener Parameter auf das Ergebnis der Kultur untersucht werden, wobei meß- und regeltechnisch möglichst schon die Bedingungen der Produktionsanlage erwünscht wären, d.h. effektive Begasung und Dosage verschiedener Fiüssigkeiten. Wie bereits ober erwähnt, würde eine derartige Parallelfermentation 96 Pumpen, 96 Regler und 16 geregelte Zuluftstrecken erfordern und ist damit technisch und wirtschaftlich praktisch nicht mehr realisierbar und erreicht trotzdem nicht, sogar wenn als Kulturgefäß Blasensäulen eingesetzt werden, die meß- und regeltechnischen Bedingungen einer Produktionsanlage. Der Trend geht zu einer weiteren Miniaturisierung und Erhöhung der Zahl der Kulturgefäße, um parallel in kürzerer Zeit mehr Ergebnisse in reproduzierbarer und quantifizierbarer Form (dokumentierbar) zu erhalten. Dies ist mit dem Stand der Technik nicht mehr realisierbar, wohl aber mit der vorliegenden Erfindung. Die Funktionsmodule können in beliebiger Größe hergestellt werden und so an die Größe des Kulturgefäßes angepasst werden, wobei die Volumen der Kulturgefäße zwischen 1 ml und 50 Kubikmeter liegen können. Bei Kulturgefäßen mit einem Flüssigkeitsvolumen von 1 ml bis 500 ml kann die gesamte Vorrichtung inkl. der Flüssigkeits- und Gasvorlagen und der Ventilsteuerung am Hals des Kulturgefäßes befestigt. Der Datenaustausch mit der Steuer EDV erfolgt über Infrarotschnittstelle. Es ist somit zum Kulturgefäß nur eine Zuleitung, bestehend aus Gaszuführung und Spannungsversorgung erforderlich. Eine weitere Miniaturisierung der Vorrichtung kann dadurch erfolgen, daß die Funktionsteile und Zuleitungen in entsprechende Werkstoffe, wie Stahl oder Kunststoffe, geätzt, geschnitten oder gespritzt werden und die Ventilfunktion durch eingelegte Dichtungen, die über Stößei betrieben werden, oder beliebige andere Miniventile realisiert werden kann. Die erfindungsgemäße Vorrichtung kann mit Einbauten im Kulturgefäß, z.B. Patentanmeldung mit dem Titel "Vorrichtung als Einsatz für Kulturgefäße zur optimierten Begasung und Dosierung von geschüttelten oder gerührten Dreiphasensystemen" (Aktenzeichen wird nachgereicht) kombiniert werden. Durch Kombination entsteht ein leistungsfähiges Kulturgefäß, das auf einfachste Weise in fast beliebigem Maßstab die komplette Meß- und Regeltechnik und die verfahrenstechnischen Parameter eines Hochleistungsfermenters wiedergibt und simulieren kann.

### Ausführungsbeispiel.

### Materalien:

Kulturgefäß: 1000 ml Erlenmeyerkolben (Enghals) mit Kappsenberg.

**Mediumszusammensetzung:**

| | |
|---|---|
| Hefeextrakt für die Mikrobiologie | 20 g/l |
| Glucose für die Mikrobiologie | 1 g/l |
| Ammoniumsulfat | 1,5 g/l |
| Kochsalz | 0,1 molar |
| Magnesiumchlorid | 0,5 g/l |
| Kaliumphosphatpuffer | 0,1 molar, pH 7,2 als Lösungsmittel |
| Olivenöl, extravirgine | 1 ml/l |

Dreiwegeventil DV1: The Lee Company, Typ LHDA12311115H
Taktventil V1, V2: The Lee Company, Typ LFVA 123021 OH
Einlaßventil EV1,EV2: The Lee Company, Typ LFVA 123021 OH
Venturidüse VD1 VD2: Spraying Systems, Typ
Belüfterdüse BD1: Spaying Systems, Typ
Ausströmerdüse AD1: Spaying Systems, Typ
Luftbehälter B1: Braun Melsungen, Einwegspritze 50 ml mit Luer Lock
Luftfilter Fl: Sartorius, Einmalsterilfilter, 0,2 um
Flüssigkeitsvorlagen: Einmalampullen, 25 ml mit Bördelkappe und Gummidichtung
Schläuche: Teflonschlauch, 1 mm Innendurchmesser Kupplungen: LuerLock
Schaumdetektion: isolierte Nadel mit Masseschluß zur Reaktionsflüssigkeit
Ventilsteuerung: Braun Melsungen DCU 3 System

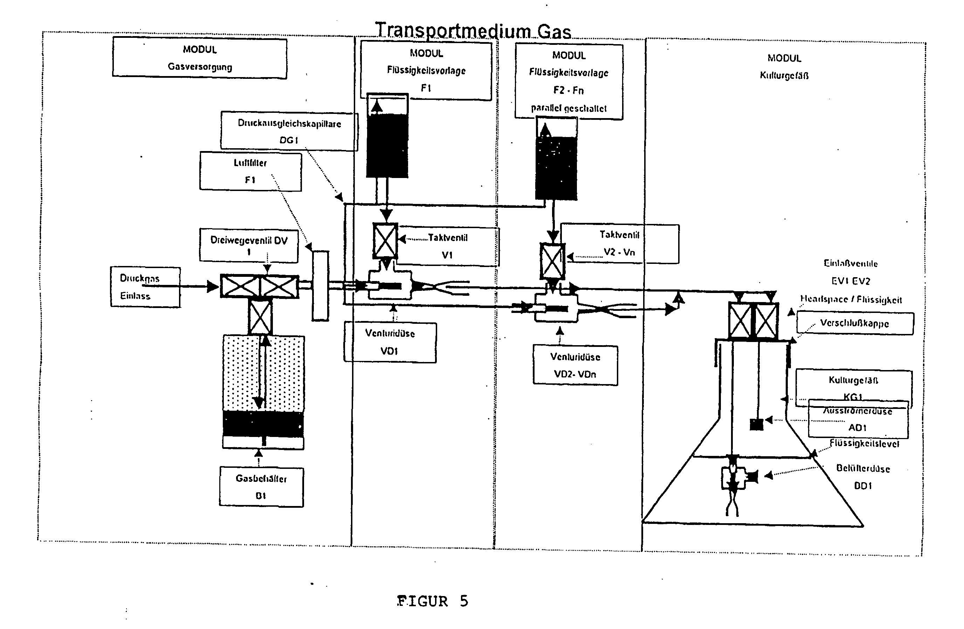

Die Medienbestandteile sind beim einschlägigen Fachhandel in gleicher Qualität erhältlich. Die Bestandteile Glucose und Magnesiumchlorid wurden als geeignete Aliquots separat sterilisiert und anschließend unter stehlen Bedingungen zugegeben. Das Kulturgefäß wurde mit 500 ml Medium befüllt und im Autoklaven sterlisiert. Die Zuleitungen zum Headspace und zur Reaktionsflüssigkeit mit den Düsen wurden durch eine Bohrung im Deckel geführt, verschlossen und ebenfalls mit dem Gefäß mitsterilisiert. Die Trennung zur erfindungsgemäßen Vorrichtung erfolgte am Ausgang der Einlaßventile. Als Flüssigkeitsvorlagen dienten jeweils 24 ml Glucoselösung (100 g/l) und 24 ml Antischaummittel (Dow Silikonöl, 10 % Suspension), die jeweils separat sterilisiert wurden. Die erfindungsgemäße Vorrichtung wurde, soweit die einzelnen Bauteile keine andere Befestigung vorsahen, gemäß Figur 5 mit Luer Lock Verbindungen und Teflonschläuchen verbunden und auf einer Arbeitsplatte befestigt. Der Leitungsteil zwischen Luftfilterausgang und Ausgang der Einlaßventile sowie die zu- und Abführungen der Flüssigkeitsvorlage wurden mit 10 m Natronlauge dekontaminiert (2h), und anschließend mit sterilem 0,1 m Phosphatpuffer pH Wert = 7,2, gespült. Nach der Sterilisation und dem Abkühlen des Kulturgefäßes erfolgte die Beimpfung mit einer Reinkultur des Mikroorganismus mit je einem Milliliter unter sterile Bedingungen. Die Reinkultur wurde aus einem Röhrchen mit E. coli, K12, erhältlich bei der deutschen Stammsammlung (DSM Hannover) und Kultivierung des Inhalts dieses Röhrchens in 10 ml Standard 1 Medium (Merck Darmstadt) bei 37 °C über 12 Stunden unter sterilen Bedingungen hergestellt. Die optische Dichte der Reinkultur betrug zum Zeitpunkt der Überimpfung 0,9 OD (546 nm). Die Vorrichtung wurde mit den Einlaßventilen mit dem Kulturgefäß und mit den Modul Gasversorgung gekoppelt. An die Flüssigkeitsvorlage 1 wurde die Glucoselösung, an die zweite das Antischaummittel angeschlossen. Die Flüssigkeitsvorlagen wurden stehend benutzt. Als Anschluß für die Drucküberlagerung wurde eine kurze, für die Flüssigkeitsentnahme eine lange Einmalinjektionsnadel benutzt, die steril durch die Gummidichtung geführt wurde. Am Drucklufteinlaß wurde Pressluft mit einem Überdruck von 0,5 bar angeschlossen. Das Volumen des Gasbehälters wurde auf 25 ml eingestellt. Die gesamte Vorrichtung und das Kulturgefäß wurden in einem Inkubator auf 37 °C temperiert. Das Kulturgefäß wurde nicht geschüttelt, da alleine der Gasstrom für ausreichende Gasversorgung der Kultur sorgte. Die Ventile der erfindungsgemäßen Vorrichtung wurden mit der Steuereinheit DCU3 verbunden und wie in Tabelle 3 gezeigt, geregelt:

### Tabelle 3

### Gasversorgung:

Taktrate 15 Füllungen und Gasströme pro Minute, entspricht einem VF von 45 oder 22,5 l Luft /h, Einlaßventil EV1 geschlossen, EV 2 offen, d.h. Gasstrom in die Reaktionsflüssigkeit

### Flüssigvorlage 1, Substrat:

Taktventil V1, geöffnet viermal pro Minute für 0,2 Sekunden, zeitgleich mit der Schaltung eines Gasstroms zum Kulturgefäß, DV1 zum Kulturgefäß hin offen, EV2 offen, entspricht einer Glucosedosage von 1 ml pro Stunde

### Flüssigvorlage 2, Antischaummittel:

Taktventil V2 normalerweise geschlossen. Wenn die Aufnehmernadel ein Schaumsignal anzeigt, läuft folgender Algorythmus ab: Einlaßventil EV2 wird geschlossen, Einlaßventil EV1 geöffnet, d.h. Headspacebegasung Start eines Timers. Ist das Schaumsignal der Aufnehmemadel nach 8 Sekunden negativ, wird das Ventil EV1 geschlossen und das Ventil EV2 geöffnet, Rückkehr zum Normalbetrieb. Liegt das Schaumsignal weiterhin an, wird zeitgleich mit jedem Takt der Gasversorgung das Taktventil V2 für 1 Sekunde geöffnet und so Antischaummittel (18,7 ml/h) in den Luftstrom der Gasversorgung gemischt. Liegt das Schaumsignal nach weiteren 16 Sekunden immer noch an, wird das Ventil EV2 zusätzlich geöffnet, um die Kultur wieder mit Gas zu versorgen. Dieser Zustand wird beibehalten, bis das Signal der Aufnehmernadel negativ ist. Danach Rückkehr in den Normalbetrieb.

Nach 24 Stunden wurde die Kultivierung der Mikroorganismen gestoppt und die optische Dichte (OD) bei 546 nm in einem Photometer bestimmt. Die OD von ca. 90 entspricht dem zu erwartenden Wert in einem Hochleistungsfermenter und überzeugte von der Leistungsfähigkeit der Vorrichtung. Die Substratvodage war zu diesem Zeitpunkt gänzlich aufgebraucht. An Antischaummittel wurde ein Verbrauch von etwa etwa 2 ml gemessen, deutlich weniger als die Menge, die ein herkömmlicher Fermenter für diesen Ansatz gebraucht hätte (etwa 12 ml, abhängig von Regelalgorithmus).

Bei der Durchführung dieses Ausführungsbeispiels waren besonders deutlich zu erkennen:
- Die kompakte, einfache Ausführungsweise der erfindungsgemäßen Vorrichtung
- Die Effektivität des"gepulsten" Begasungssystems in Kombination mit der Belüfterdüse
- Die entstehenden, extrem feinen Gasblasen
- Die kurzen Mischzeiten des Systems
- Die Leistungsfähigkeit der Schaumbekämpfung durch den erfindungsgemäßen Aufbau
- Die exakte gleichmäßige Dosierung der Flüssigkeiten.

Es soll nochmals darauf hingewiesen werden, das diese Ergebnisse, die denen eines Hochleistungsfermeners entsprechen, ohne Schütteln oder Rühren des Kulturgefäßes erreicht wurden. In Kombination mit Einsätzen, durch Schüttler oder Rührer, läßt sich die Leistungsfähigkeit weiter steigern.

Zusammenfassen ist insofern auch offenbart ein Verfahren zur dosierten Zugabe von einem oder mehreren Fluiden oder Fluidgemischen in ein oder mehrere Kulturgefäße, gekennzeichnet durch die Verwendung von mindestens einem Carrierfluid, das quantitativ, diskontinuierlich aus einem unter Druck stehenden Vorratsgefäß mit definiertem Innenvolumen über ein Taktventil entnommen wird.

Weiterbildung sind gekennzeichnet in beliebiger Kombination durch die Verwendung eines Carriergases oder einer Carriergasmischung, durch die Verwendung einer Carrierflüssigkeit oder einer Carrierflüssigkeitsmischung, dadurch, dass das/die zu dosierende(n) Fluid(e) dem/den Carrierfluid(en) über eine oder mehrere Venturidüsen dosiert zugemischt werden, dadurch, dass die Zuleitung ins Reaktionsmedium im Kulturgefäß zur besseren Durchmischung über eine Venturidüse erfolgt, dadurch, dass ein Filter o.ä. am Seiteneinlass der Venturidüse im Reaktionsmedium den Eintritt von Mikroorganismen in die Düse verhindert, durch eine Zuführung in den Headspace des Kulturgefäßes, durch eine Vernebelungseinrichtung wie z.B. eine Ausströmdüse am Eintritt in den Headspace, durch eine Umschaltung der Zuführung ins Kulturgefäß von der Zuführung ins Reaktionsmedium zur Zuführung in den Headspace und zurück, dadurch, dass das Carriergas oder die Carriergasmischung aus einem Gasbehälter unter Überdruck entnommen wird, dadurch, dass der Druck im Gasbehälter während des Prozesses nach einem oder mehreren Entnahmen einmal oder mehrfach über eine Zuleitung wieder erhöht wird, dadurch, dass diese dem Carrierfluid über verschiedene Venturidüsen in Serien- oder Parallelschaltung, bevorzugt in Parallelschaltung, zugleich oder in beliebiger Reihenfolge zugemischt werden.

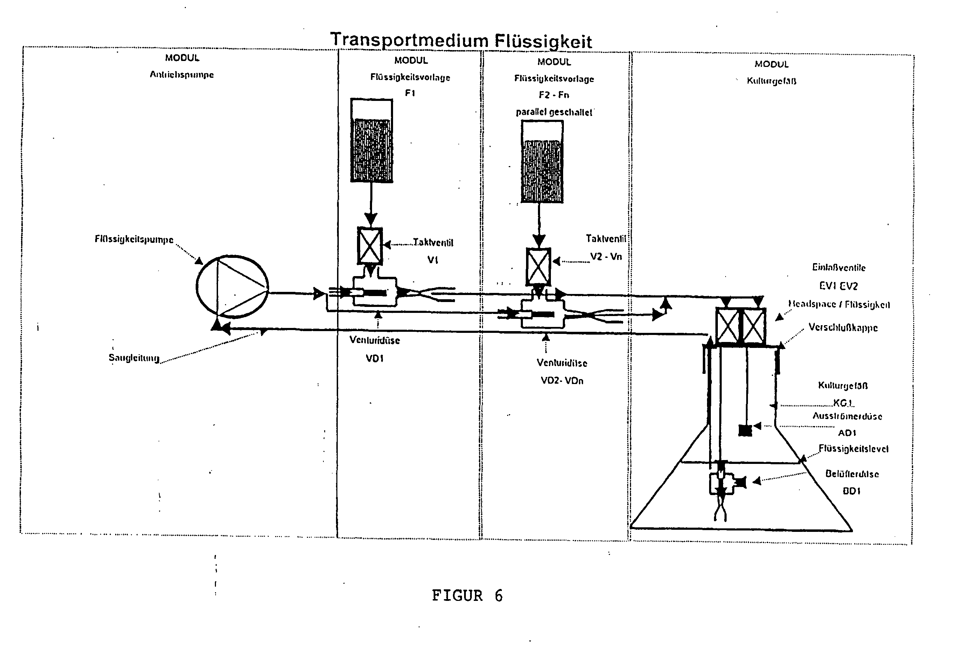

Weiterhin ist insofern offenbart eine Vorrichtung zur Dosierung von Gasen oder Flüssigkeiten oder Gemischen daraus zum Einsatz an Kulturgefäßen für biologische und (bio)chemische Reaktionen, welche dadurch gekennzeichnet ist, daß die Vorrichtung aus mindestens einem Modul Gasversorgung gemäß Figur 5 oder einem Modul Antriebspumpe gemäß Figur 6 zur Erzeugung eines Carrierfluids, einer Venturidüse mit Taktventil und Flüssigkeitsvorlage mit einer Druckausgleichskapillare, oder einer Dosiervorlage für Gase, und einer Zuleitung, die in der Reaktionsflüssigkeit oder im Headspace des Kulturgefäßes mündet, besteht. Das Modul Gasversorgung oder das Modul Antriebspumpe erzeugt einen Strom von Carrierfluid, kontinuierlich oder diskontinuierlich, über die Zuleitung zum Kulturgefäß hin. Weiterbildung sind in beliebiger Kombination dadurch gekennzeichnet, dass das Volumen des Gasbehälters B1 zwischen 1% und 40% des Volumens des Kulturgefäßes beträgt, dass das Volumen des Gasbehäiters durch einen Stempel im Bereich von 0% bis 100% des Gesamtvolumens variiert werden kann, daß die Taktrate des/der Ventils(e) auf den "gas hold up" der Gasblasen im Kulturgefäß abgestimmt werden kann, bevorzugt mit diesem identisch ist und die Menge des durchgesetzten Gases durch Verstellen des Stempels gemäß Anspruch 3 erfolgtdaß mindestens 1 Modul Flüssigkeitsvorlage oder Gasvorlage gemäß Zeichnung 1 zwischen Dreiwegeventil und Kulturgefäß geschaltet wird, daß das Modul Flüssigkeitsvorlage aus mindestens einer Venturidüse und einem Flüssigkeitsbehälter besteht und der Druckausgleich entweder über die Druckausgleichskapillare zum Gasbehälter oder durch eine Verbindung mit der Außenatmosphäre erfolgen kann, daß die Flüssigkeitsvorlagen beliebig, hängend, stehend, liegend zur Vorrichtung angebracht sind und immer einen Luftraum von mindestens 2% des Gesamtvolumens aufweisen, in den der Druckausgleichs erfolgen kann, daß die Gasdosiervorlage mindestens aus einem Gaseinlaß, einem Dreiwegeventil oder 2 Ventilen, einem Gasbehälter mit variablen Innenvolumen (analog Anspruch 15) besteht und eine Ventilöffnung mit einem Seiteneinlaß einer Venturidüse verbunden ist, daß die Abmessungen der Komponenten der Vorrichtung und somit die erfindungsgemäßen Eigenschaften an Kulturgefäße von 1 Milliliter bis 50 Liter Volumen angepasst werden kann, daß mit der Vorrichtung bessere Durchmischungen und Austauschraten mit der Reaktionsflüssigkeit erzielt werden, so daß auf ein Schütteln oder Rühren des Kulturgefäßes, abhängig von der Art der Kultivierung, verzichtet werden kann, daß durch die Art der diskontinuierlichen Begasung und des Gaseintrags die Neigung zur Schaumbildung der Reaktionsflüssigkeit reduziert wird, daß durch die Kombination Gas/Flüssigkeitsdosierung, ob in die Reaktionsflüssigkeit oder in den Headspace des Kulturgefäßes, eine kürzere Mischzeit mit der Reaktionsflüssigkeit erreicht wird und somit Konzentrationsgradienten (z.B. Substrat) minimiert werden, daß insbesondere durch die Dosierung in den Headspace des Kulturgefäßes eine effektivere Ausnutzung der Eigenschaften der dosierten Flüssigkeiten erfolgt, daß mit dem Einsatz der Vorrichtung der Verbrauch von Antischaummitteln minimiert werden kann. Hierfür wird kurzzeitig der Gasfluß in die Reaktionsflüssigkeit abgeschaltet und auf die Ausströmerdüse der Head Space Dosierung geschaltet. Die ausströmende Luft "bläst" den Schaum auf die Flüssigkeitsoberfläche zurück. In den meisten Fällen genügt dieser Effekt, um die Schaumbildung zu reduzieren (im negativen Fall erfolgt eine Dosierung, die in Kombination mit diesem Verfahren den Verbrauch von Antischaummittein auf 10 % gegenüber dem Stand der Technik senken kann), daß so die dosierten Flüssigkeiten oder eine Kombination mehrerer einen Effekt auf die Reaktionsflüssigkeit haben können, z.B. durch zeitabhängige Zugabe von Phagen zu Bakerienkulturen oder Wachstumsfaktoren zu Zellkulturen oder pH Regelung mittels C02, daß die in dieser Weise dosierten Gase zur Schaffung einer künstlichen Atmosphäre in head space des Kulturgefäßes, z.B. anaerobe Athmosphäre oder mit CO2 angereichert, mit definierter Zusammensetzung benutzt werden, daß die Vorrichtung nur mit einer Stromversorgung und Transportmedienversogung verbunden ist und alle meß- und regeltechnischen Parameter mit der Kontroll EDV über Infrarotschnittsteile ausgetauscht werden. In aller Allgemeinheit meint dies einen Bioreaktor zur Kultivierung von Zellen, mit einem Kulturgefäß, mit einer oder mehreren Gasversorgungen und/oder einer oder mehreren Flüssigkeitsvorlagen sowie Zuführungseinrichtungen für Gase und/oder Flüssigkeiten, mittels welcher Gase und/oder Flüssigkeiten dem Kulturgefäß zuführbar sind, wobei zwischen der Zuführungseinrichtung und der Gasversorgung bzw. Flüssigkeitsvorlage eine Mischvorrichtung, insbesondere eine Venturidüse, zur Mischung von Gas und/oder Flüssigkeit aus der Gasversorgung bzw. Flüssigkeitsvorlage geschaltet ist. Gas und Flüssigkeit können zu einem Aerosol gemischt werden. Zwischen der Mischvorrichtung und der Gasversorgung bzw. der Flüssigkeitsvorlage können ansteuerbare Ventile, insbesondere Taktventile geschaltet sein. Weiterhin meint dies ein Verfahren zum Betrieb eines vorstehenden Bioreaktors, wobei ein Gas oder eine Flüssigkeit als Carrierfluid eingesetzt wird, wobei dem Carrierfluid in der Mischvorrichtung ein Gas oder eine Flüssigkeit zugemischt wird, und wobei die Mengenverhältnisse der miteinander gemischten Fluide definiert sind und gesteuert oder geregelt werden. Die gemischten Fluide können in definiertem Mengenstrom dem Kulturgefäß zugeführt werden.

## Patentansprüche

1. Einsetzbare Apparatur für Kulturgefäße zur Begasung und/oder Dosierung, bestehend aus mindestens einem Wirbelstrombrecher, mindestens einem Fixierungsteil und mindestens einer Gas- oder Flüssigkeitszuleitung, **gekennzeichnet dadurch, daß** die Apparatur ihre endgültige Form erst nach dem Einsetzen in das Gefäß durch Aufblasen oder Auffalten vor der Verwendung erreicht.

2. Apparatur mit Kulturgefäß zur Begasung und/oder Dosierung, bestehend aus mindestens einem Wirbelstrombrecher, mindestens einem Fixierungsteil und mindestens einer Gas- oder Flüssigkeitszuleitung, **gekennzeichnet dadurch, daß** die Apparatur ihre endgültige Form zusammen mit dem Gefäß durch Aufblasen oder Auffalten vor der Verwendung erreicht.

3. Apparatur nach Anspruch 1 oder 2, **gekennzeichnet durch** ein Fixierungsteil in Form eines Fixierringes oder eines der Gefäßform angepassten Doppel-T-Fixierstückes, das sich nach Erreichen der endgültigen Form bevorzugt in der Nähe des Gefäßbodens befindet und die Apparatur so fixiert.

4. Apparatur nach einem der vorigen Ansprüche, bestehend aus dehnbaren oder nicht dehnbaren synthetischen oder natürlichen Polymeren.

5. Apparatur nach einem der vorigen Ansprüche, wobei das Polymer ganz oder in Teilen nur Substanzen in bestimmten Molekülgrössenbereichen durchlässt, insbesondere zwischen dem Innenraum der Apparatur und der Reaktionsflüssigkeit.

6. Apparatur nach einem der vorigen Ansprüche, bestehend aus nur einem Folienteil nach Art eines geformten Ballons.

7. Apparatur nach einem der Ansprüche 1-5, bestehend aus mehreren Folienteilen, die formgebend miteinander verklebt oder verschweißt werden.

8. Apparatur nach einem der vorigen Ansprüche, **dadurch gekennzeichnet, daß** durch die Apparatur auch die Wandungen und die Form eines Kulturgefäßes gebildet wird und so ein aufblasbares oder auffaltbares Kulturgefäß entsteht.

9. Apparatur nach einem der vorigen Ansprüche, **gekennzeichnet dadurch, daß** die endgültige Form durch kontinuierliches Durchströmen mit einer Flüssigkeit oder einem Gas unter Überdruck von mindestens 0,2 bar erreicht wird.

10. Apparatur nach einem der vorigen Ansprüche, **gekennzeichnet durch** die Fixierung von einem oder mehreren Wirbelstrombrechem je **durch** einen oder mehrere Stege.

11. Apparatur nach einem der vorigen Ansprüche, **gekennzeichnet dadurch, daß** die Wirbelstrombrecher gelocht sind und/oder einen Abstand zur Gefäßwand von 1-20% ihres Durchmessers haben.

12. Apparatur nach einem der vorigen Ansprüche, **gekennzeichnet durch** eine Versteifung des der Gefäßwand zugewandten Teils **durch** eine eingeschweisste Schiene.

13. Apparatur nach einem der vorigen Ansprüche, **gekennzeichnet durch** eine Armatur zum Einführen von Messsonden, die **durch** eine für zu bestimmende Substanzen durchlässige Membran von der Reaktionsflüssigkeit abgetrennt ist.

14. Apparatur nach einem der vorigen Ansprüche, **gekennzeichnet durch** eine Fixierungsmöglichkeit für die Armatur, insbesondere an den Stegen.

15. Apparatur nach einem der vorigen Ansprüche, **gekennzeichnet durch** darin eingeschweißte Kanäle oder darin eingelegte Schläuche zur Verbindung der Reaktionsflüssigkeit im Gefäß und/oder des darüberliegenden Gasraumes im Gefäß nach außen.

16. Verwendung einer Apparatur nach einem der vorigen Ansprüche in einem wiederverwendbaren Einsatz zur Fixierung im Kulturgefäß.

17. Verwendung von mehreren gleichen oder verschiedenen Apparaturen nach einem der Ansprüche 1-15 in einem Kulturgefäß.

18. Verwendung einer Apparatur nach einem der Ansprüche 1-15 in einem oder als Kulturgefäß mit einem Volumen von 2-20000 ml.

19. Verwendung nach Anspruch 18 in einem oder als Kulturgefäß für biologische und/oder biochemische Reaktionen, bevorzugt in Zwei- oder Dreiphasensystemen, bevorzugt zur Fermentation von Mikroorganismen, bevorzugt unter sterilen Bedingungen.

20. Verwendung einer Apparatur nach einem der Ansprüche 1-15 zur Zu- und Abführung von Flüssigkeiten und/oder Gasen in und aus der Reaktionsflüssigkeit sowie in den und aus dem darüber liegenden Gasraum.

21. Verwendung einer Apparatur nach einem der Ansprüche 1-15 zur Temperierung der Reaktionsflüssigkeit mit dem durch geleiteten Gas oder der durchgeleiteten Flüssigkeit.

22. Verwendung einer Apparatur nach einem der Ansprüche 1 bis 15 zur Herstellung eines Bioreaktors, mit einem durch Reaktorwandungen begrenzten Reaktorraum sowie mit einer Reaktoröffnung, optional mit durch die Reaktoröffnung greifenden Mitteln zur Zufuhr von Gasen und/oder Flüssigkeiten, zur Probenentnahme, zur Zufuhr von Mikroorganismen oder Zellen, zur Einführung von Messsonden, zur Zugabe von Additiven oder Sterilisationsmitteln und/oder zum Entleeren des Bioreaktors, mit Mitteln zum Verschluss der Reaktoröffnung, wobei mindestens eine Apparatur nach einem der Ansprüche 1 bis 14, enthaltend mindestens einen aufblasbaren Wirbelstrombrecher, im Reaktorraum eingerichtet ist.

23. Verwendung nach Anspruch 22, wobei der aufblasbare Wirbelstrombrecher eine von den Reaktorwandungen separate bauliche Einheit ist und im kollabierten Zustand durch die Reaktoröffnung einführbar und dann aufblasbar ist.

24. Verwendung nach Anspruch 22, wobei die Reaktorwandungen aus einem flexiblen Material gebildet sind, und wobei die Reaktorwandungen mit dem Wirbelstrombrecher eine bauliche Einheit bilden.

25. Verwendung nach Anspruch 24, wobei eine Mehrzahl von Wirbelstrombrecher eingerichtet sind, die in aufgeblasenem Zustand so gegeneinander stabilisiert sind, daß die Reaktorwandungen einen mechanisch stabilen Reaktionsraum bilden.

26. Verwendung nach einem der Ansprüche 22 bis 25, wobei die Mittel gemäß Anspruch 21 als mit dem Wirbelstrombrecher verbundene Schlauchleitungen ausgebildet sind.

## Claims

1. Applicable apparatus for culture vessels for introduction of gas and/or dosing, consisting of at least one eddy current cup, at least one fixing portion and at least one gas or liquid feed line, **characterized in that** the apparatus arrives at its final shape after inserting the vessel not until it is inflated or unfolded prior to its use.

2. Apparatus including a culture vessel for introduction of gas and/or dosing, consisting of at least one eddy current cup, at least one fixing portion and at least one gas or liquid feed line, **characterized in that** the apparatus arrives at its final shape in conjunction with the vessel by inflation or unfolding prior to its use.

3. Apparatus according to claim 1 or 2, **characterized by** a fixing portion of a fixing ring or of a double-T fixing piece that, after having arrived at the final shape, is preferably located near the bottom of the vessel and is thus fixing the apparatus.

4. Apparatus according to any of the preceding claims, consisting of elastic or non-elastic synthetic or natural polymers.

5. Apparatus according to any of the preceding claims, the polymer transmitting totally or in part only substances within specific molecule size ranges, in particular between the inner space of the apparatus and the liquid reactant.

6. Apparatus according to any of the preceding claims, consisting only of a foil portion of a shaped balloon-type.

7. Apparatus according to any of claims 1 - 5, consisting of plural foil portions that are adhered or welded together in a shaping manner.

8. Apparatus according to any of the preceding claims, **characterized in that** the apparatus also forms the walls and the shape of a culture vessel and thus, an inflatable or unfoldable culture vessel is formed.

9. Apparatus according to any of the preceding claims, **characterized in that** the final shape is achieved by a liquid or a gas at an excess pressure of at least 0.2 bar continuously flowing through.

10. Apparatus according to any of the preceding claims, **characterized by** the fixation of one or more eddy current cups by one or more stays, respectively.

11. Apparatus according to any of the preceding claims, **characterized in that** the eddy current cups are perforated and/or have a distance to the vessel wall of 1 - 20% of their diameter.

12. Apparatus according to any of the preceding claims, **characterized by** a stiffening of the part facing the vessel wall through a welded rail therein.

13. Apparatus according to any of the preceding claims, **characterized by** a mounting for insertion of measuring probes that is separated from the reactive liquid by a diaphragm permeable to substances to be identified.

14. Apparatus according to any of the preceding claims, **characterized by** a fixation setting for the mounting, in particular at the stays.

15. Apparatus according to any of the preceding claims, **characterized by** welded channels therein or inlaid hoses therein for communication of the reactive liquid in the vessel and/or of the gaseous space above in the vessel to the exterior.

16. Use of an apparatus according to any of the preceding claims in a reusable insert for fixation in the culture vessel.

17. Use of plural similar or different apparatuses according to any of claims 1 - 15 within a culture vessel.

18. Use of an apparatus according to any of claims 1 - 15 within or as a culture vessel having a capacity of 2 - 20000 ml.

19. Use according to claim 18 within or as a culture vessel for biological and/or biochemical reactions, preferably in two- or three-phase systems, preferably for fermentation of microorganisms, preferably under sterile conditions.

20. Use of an apparatus according to any of claims 1 - 15 for feed and discharge of liquids and/or gases both in and out of the reactant liquid and in and out of the above gaseous space.

21. Use of an apparatus according to any of claims 1 - 15 for tempering the reactant liquid by the passage of the gas or the passage of the liquid.

22. Use of an apparatus according to any of claims 1 - 15 for producing a bioreactor including a reactor space defined by the reactor walls and a reactor opening, optionally including means engaging through the reactor opening for feeding of gases and/or liquids, for sampling, for supplying microorganisms or cells, for inserting measuring probes, for adding additives or sterilising agents and/or for draining the bioreactor, including means for closing the reactor opening, at least one apparatus according to any of claims 1 - 14 including at least one eddy current cup being arranged in the reactor space.

23. Use according to claim 22, wherein the inflatable eddy current cup is a separate structural unit and can be inserted in its collapsed state through the reactor opening and then be inflated.

24. Use according to claim 22, wherein the reactor walls are formed of a flexible material, and wherein the reactor walls form a structural unit with the eddy current cup.

25. Use according to claim 24, wherein a plurality of eddy current cups are arranged, which are stabilised against each other in such a manner that the reactor walls form a mechanically robust reaction space.

26. Use according to any of claims 22 to 25, wherein the means according to claim 21 are formed as hose lines connected to the eddy current cup.

## Revendications

1. Appareillage insérable pour récipients de culture destinés au gazage et/ou au dosage, composé d'au moins un broyeur tourbillonnaire, d'au moins une partie de fixation et d'au moins une conduite d'alimentation en gaz ou en liquide, **caractérisé en ce que** l'appareillage n'atteint sa forme définitive qu'après l'insertion dans le récipient par gonflage ou par déploiement avant l'utilisation.

2. Appareillage avec récipient de culture destiné au gazage et/ou au dosage, composé d'au moins un broyeur tourbillonnaire, d'au moins une partie de fixation et d'au moins une conduite d'alimentation en gaz ou en liquide, **caractérisé en ce que** l'appareillage atteint sa forme définitive avec le récipient par gonflage ou par déploiement avant l'utilisation.

3. Appareillage selon la revendication 1 ou 2, **caractérisé par** une partie de fixation, sous la forme d'un anneau de fixation ou d'une pièce de fixation en double T adaptée à la forme de récipient et qui, une fois atteinte la forme définitive, se trouve de préférence près du fond du récipient et fixe ainsi l'appareillage.

4. Appareillage selon une des revendications précédentes, composé de polymères synthétiques ou naturels extensibles ou non extensibles

5. Appareillage selon une des revendications précédentes, le polymère ne laissant passer totalement ou de façon partielle que des substances dans des plages de dimension moléculaire définies, en particulier entre l'espace intérieur de l'appareillage et le liquide de réaction.

6. Appareillage selon une des revendications précédentes, composé de seulement une partie de film à la façon d'un ballon formé.

7. Appareillage selon une des revendications 1-5, composé de plusieurs parties de film qui sont assemblées les unes aux autres par collage ou soudage pour réaliser une forme.

8. Appareillage selon une des revendications précédentes, **caractérisé en ce que**, sous l'action de l'appareillage, les parois et la forme d'un récipient de culture sont également formées et **en ce qu'**il en résulte un récipient de culture gonflable ou pouvant être déployé.

9. Appareillage selon une des revendications précédentes, **caractérisé en ce que** la forme définitive est atteinte par la réalisation d'un flux continu avec un liquide ou un gaz en présence d'une surpression d'au moins 0,2 bar.

10. Appareillage selon une des revendications précédentes, **caractérisé par** la fixation d'un ou de plusieurs broyeurs tourbillonnaires respectivement par une ou plusieurs entretoises.

11. Appareillage selon une des revendications précédentes, **caractérisé en ce que** les broyeurs tourbillonnaires sont perforés et/ou présentent un écart à la paroi de récipient qui est égal à 1 à 20 % de leur diamètre.

12. Appareillage selon une des revendications précédentes, **caractérisé par** un renforcement, par un rail soudé, de la partie tournée vers la paroi de récipient.

13. Appareillage selon une des revendications précédentes, **caractérisé par** une garniture destinée à introduire des sondes de mesure qui est séparée du liquide de réaction par une membrane perméable à des substances à définir.

14. Appareillage selon une des revendications précédentes, **caractérisé par** une possibilité de fixation de la garniture, en particulier sur les entretoises.

15. Appareillage selon une des revendications précédentes, **caractérisé par** des canaux qui y sont soudés ou des tuyaux qui y sont insérés pour le raccordement du liquide de réaction dans le récipient et/ou de l'espace de gaz placé au-dessus dans le récipient vers l'extérieur.

16. Utilisation d'un appareillage selon une des revendications précédentes dans un emploi réutilisable pour la fixation dans le récipient de culture.

17. Utilisation de plusieurs appareillages identiques ou différents selon une des revendications 1-15 dans un récipient de culture.

18. Utilisation d'un appareillage selon une des revendications 1 - 15 dans un récipient de culture ou en tant que récipient de culture avec un volume de 2-20 000 ml.

19. Utilisation selon la revendications 18 dans un récipient de culture ou en tant que récipient de culture pour des réactions biologiques et/ou biochimiques, de préférence dans des systèmes à deux ou à trois phases, de préférence pour la fermentation de microorganismes, de préférence dans des conditions stériles.

20. Utilisation d'un appareillage selon une des revendications 1 - 15 pour l'alimentation et l'évacuation de liquides et/ou de gaz vers ou à partir du liquide de réaction ainsi que vers ou à partir de l'espace de gaz placé au-dessus.

21. Utilisation d'un appareillage selon une des revendications 1 - 15 pour l'équilibrage de température du liquide de réaction avec le gaz transporté ou le liquide transporté.

22. Utilisation d'un appareillage selon une des revendications 1 - 15 pour la fabrication d'un bioréacteur, avec un espace de réacteur limité par des parois de réacteur ainsi qu'avec une ouverture de réacteur, optionnellement avec des moyens pénétrant dans l'ouverture de réacteur pour l'amenée de gaz et/ou de liquides, pour le prélèvement d'échantillons, pour l'amenée de microorganismes ou de cellules, pour l'introduction de sondes de mesure, pour l'ajout d'additifs ou d'agents de stérilisation et/ou pour le vidage du bioréacteur, avec des moyens pour l'obturation de l'ouverture de réacteur, au moins un appareillage selon une des revendications 1 à 14, contenant au moins un broyeur tourbillonnaire gonflable, étant aménagé dans l'espace de réacteur.

23. Utilisation selon la revendication 22, le broyeur tourbillonnaire gonflable étant une unité constructive séparée des parois de réacteur et pouvant, dans l'état aplati, être introduit à travers l'ouverture de réacteur et puis gonflé.

24. Utilisation selon la revendication 22, les parois de réacteur étant formées à partir d'un matériau flexible, et les parois de réacteur formant une unité constructive avec le broyeur tourbillonnaire.

25. Utilisation selon la revendication 24, une pluralité de broyeurs tourbillonnaires étant aménagés qui, dans l'état gonflé, sont stabilisés les uns contre les autres de telle façon que les parois de réacteur forment un espace de réaction mécaniquement stable.

26. Utilisation selon une des revendications 22 à 25, les moyens selon la revendication 21 étant constitués en tant que tuyaux souples raccordés au broyeur tourbillonnaire.
